(12) **EUROPEAN PATENT SPECIFICATION**

(45) Date of publication and mention
of the grant of the patent:
**09.10.2024 Bulletin 2024/41**

(21) Application number: **19198781.7**

(22) Date of filing: **20.09.2019**

(51) International Patent Classification (IPC):
**A61B 8/08** *(2006.01)* **G06T 7/00** *(2017.01)*
**G06T 7/246** *(2017.01)*

(52) Cooperative Patent Classification (CPC):
**A61B 8/0883; A61B 8/485; A61B 8/5223;**
**G06T 7/0016; G16H 50/30;** G06T 2207/10016;
G06T 2207/10132; G06T 2207/20081;
G06T 2207/20084; G06T 2207/30048

(54) **APPARATUS AND COMPUTER PROGRAM**

VORRICHTUNG UND COMPUTERPROGRAMM

APPAREIL ET PROGRAMME INFORMATIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **21.09.2018 JP 2018177255**
**18.09.2019 JP 2019168987**

(43) Date of publication of application:
**25.03.2020 Bulletin 2020/13**

(73) Proprietor: **Canon Medical Systems Corporation**
**Otawara-shi, Tochigi 324-0036 (JP)**

(72) Inventor: **ABE, Yasuhiko**
**Otawara-shi, Tochigi (JP)**

(74) Representative: **Marks & Clerk LLP**
**15 Fetter Lane**
**London EC4A 1BW (GB)**

(56) References cited:
**WO-A1-2005/078631 WO-A1-2006/083569**
**WO-A2-2005/001769 US-A1- 2006 074 315**

**Description**

FIELD

Embodiments described herein related generally to an apparatus and a computer program.

BACKGROUND

A wall motion analysis technique is known by which wall motion of the heart is analyzed by using time-series medical image data taken of the heart. For example, with ultrasound imaging, there is a method by which speckle tracking is performed on time-series ultrasound image data, so as to calculate motion information from strain or displacements of the cardiac wall.

As a symptom of cardiac diseases, it is known that local abnormality in the wall motion called hypokinesis appears in an ischemic state. For this reason, various techniques for diagnosing such an abnormality in the wall motion have been proposed by which a characteristic obtained from waveform information of strain is defined as an index value or by which an index image is displayed by mapping brightness values corresponding to the index value.

However, absolute values of strain vary among patients (examined subjects) and among regions of the heart. It is therefore not robust to identify whether or not the hypokinesis is occurring in the wall motion by using a peak value. Further, the characteristic in the waveform information found in the ischemic state may also be found in healthy people in some situations. For this reason, defining the index value on the basis of the characteristic has a low degree of specificity. Accordingly, it is difficult to use the existing techniques for identifying local abnormality in the wall motion with a high level of precision and in a robust manner.

WO2005/001769 relates to systems and methods for automated assessment of regional myocardial function using wall motion analysis methods that analyze various features/parameters of patient information (image data and non-image data) obtained from medical records of a patient. For example, a method for providing automatic diagnostic support for cardiac imaging generally comprises obtaining image data of a heart of a patient, obtaining features from the image data of the heart, which are related to motion of the myocardium of the heart, and automatically assessing regional myocardial function of one or more regions of a myocardial wall using the obtained features.

Summary of Invention

In a first aspect, an apparatus is provided as recited in claim 1.

The determining unit may determine the abnormality in the first local region of the first patient, by inputting the plurality of pieces of first medical information of the first patient to a trained model trained by using, with respect to hearts of a plurality of second patients, a plurality of pieces of second medical information indicating wall motion parameters in a second local region and in a second global region and abnormality determination results with respect to the second local region.

The wall motion parameters may include pieces of information that are of mutually-different types with respect to at least one selected from among: temporal phases before and after either a load or medical treatment; positions in a wall thickness direction; and heart motion directions, and the plurality of pieces of second medical information may include information that is of same types as the plurality of pieces of first medical information.

Each of the first global region and the second global region may correspond to one of: an entire left ventricle in a three-dimensional image; and an entire region of interest in a two-dimensional cross-sectional image; the first local region may correspond to at least one of a plurality of regions obtained by dividing the first global region into sections while using a predetermined dividing method; and the second local region correspond to at least one of a plurality of regions obtained by dividing the second global region into sections while using a predetermined dividing method.

The wall motion parameters may include information about at least one of strain and a displacement.

The temporal phases may include information about at least one of: a time period before a load; and a recovery time period.

The positions in the wall thickness direction may include either information about at least one selected from among an inner layer, a middle layer, and an outer layer; or information about at least one selected from among an inner half, an outer half, and a total of a wall thickness.

The motion directions may include information about at least one selected from among: a longitudinal direction, a circumferential direction, a radial direction, and an area size of a plane positioned parallel to an inner myocardial membrane surface.

Information about the wall motion parameter in the first global region and the second global region may further include information indicating time-series changes regarding changes in volume.

The trained model may be constructed on a basis of one of a support vector machine and a neural network.

The plurality of types of index values may include at least a Strain Imaging Diastolic Index (SI-DI) and a Post-systolic Strain Index (PSI).

The output controlling unit causes the determination result of the abnormality with respect to the first local region to be displayed while being superimposed on one of an ultrasound image and a polar map.

The pieces of first medical information and the pieces of second medical information may be pieces of information normalized in a time direction.

The determination result of the abnormality with respect to the first local region may be information indicating whether or not the first local region is an ischemic region.

The pieces of first medical information may be pieces of waveform information indicating time-series changes in the wall motion parameters in the first local region and in the first global region and the pieces of second medical information may be pieces of waveform information indicating time-series changes in the wall motion parameters in the second local region and in the second global region.

The abnormality determination results with respect to the second local region may be pieces of information obtained as a result of a human being identifying whether or not abnormality is present in wall motion.

The obtaining unit may obtain the plurality of pieces of first medical information from an ultrasound image.

The apparatus may be at least one of an ultrasound diagnosis apparatus, a medical image diagnosis apparatus, a medical information processing apparatus, and a medical information processing system.

In an embodiment, the apparatus comprises a learning unit configured to construct a trained model by performing machine learning by using, with respect to hearts of a plurality of patients, a plurality of pieces of medical information indicating wall motion parameters in a local region and in a global region and abnormality determination results with respect to the local region.

In a further aspect, a program is provided that is configured to cause a computer to execute each feature of the apparatus.

BRIEF DESCRIPTION OF THE DRAWINGS

FIG. 1 is a block diagram illustrating an exemplary configuration of an ultrasound diagnosis apparatus according to a first embodiment;

FIG. 2 is a block diagram illustrating an exemplary configuration of a medical information processing apparatus according to the first embodiment;

FIG. 3 is a drawing illustrating processes in a learning mode and an operation mode performed by the ultrasound diagnosis apparatus and the medical information processing apparatus according to the first embodiment;

FIG. 4 is a drawing for explaining a group of waveform information for a machine learning purpose according to the first embodiment;

FIG. 5 is a drawing for explaining an example of the group of waveform information according to the first embodiment;

FIG. 6 is a drawing for explaining abnormality determination results for the machine learning purpose according to the first embodiment;

FIG. 7 is a drawing for explaining an example of an output by an output controlling function according to the first embodiment;

FIG. 8 is a drawing for explaining another example of the output by the output controlling function according to the first embodiment;

FIG. 9 is a flowchart illustrating a processing procedure performed by the medical information processing apparatus according to the first embodiment;

FIG 10 is a flowchart illustrating a processing procedure performed by the ultrasound diagnosis apparatus according to the first embodiment;

FIG. 11 is a block diagram illustrating an exemplary configuration of an ultrasound diagnosis apparatus according to a second embodiment;

FIG. 12 is a chart for explaining index values calculated by a calculating function according to the second embodiment;

FIG. 13 is a table for explaining an example of an output by the output controlling function according to the second embodiment; and

FIG. 14 is a block diagram illustrating an exemplary configuration of a medical information processing system according to another embodiment.

DETAILED DESCRIPTION

It is an object of the present disclosure to provide an apparatus, a medical information processing apparatus, and a computer program product capable of identifying local abnormality in the wall motion with a high level of precision and in a robust manner.

A medical image diagnosis apparatus according to an embodiment includes processing circuitry. The processing circuitry is configured to obtain a plurality of pieces of first medical information indicating wall motion parameters in a first local region and in a first global region with respect to the heart of a first examined subject (hereinafter "first patient"). The processing circuitry is configured to determine abnormality in the first local region on the basis of the plurality of pieces of first medical information.

Exemplary embodiments of an apparatus, a medical information processing apparatus, and a computer program product will be explained below, with reference to the accompanying drawings. The embodiments described herein are not limited to the description presented below. It is possible to combine each of the embodiments described herein with any of the other embodiments or a conventional technique, as long as no conflict occurs in the processing.

First Embodiment

An ultrasound diagnosis apparatus and a medical information processing apparatus according to a first embodiment will be explained. The ultrasound diagnosis apparatus according to the first embodiment includes a trained model and is capable of identifying local abnormality in the wall motion by using the trained model with a high level of precision and in a robust manner. Further, the medical information processing apparatus according to the first embodiment is configured to construct the trained model included in the ultrasound diagnosis apparatus. In the first embodiment, the example will be explained in which the ultrasound diagnosis apparatus includes the trained model; however, possible embodiments are not limited to this example. Other embodiments in which an apparatus other than the ultrasound diagnosis apparatus includes the trained model will be explained later.

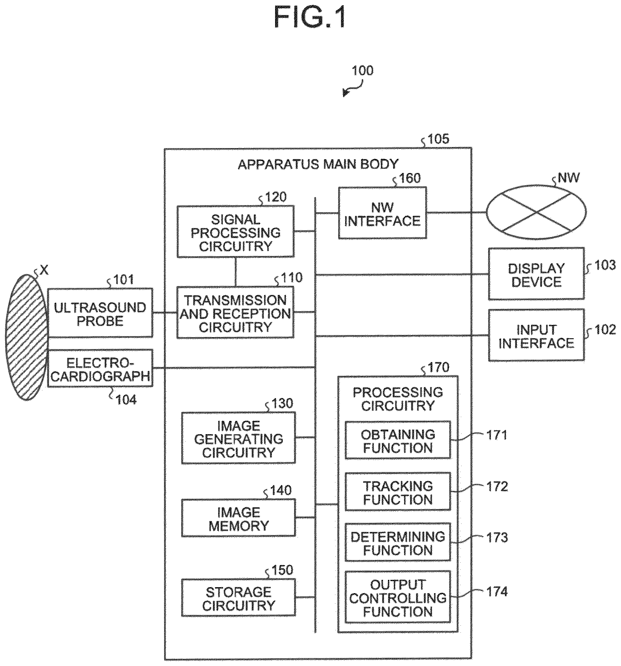

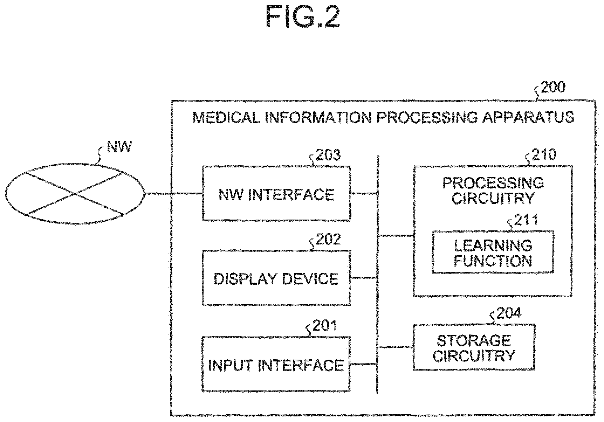

Configurations of the ultrasound diagnosis apparatus and the medical information processing apparatus according to the first embodiment will be explained, with reference to FIGS. 1 and 2. FIG. 1 is a block diagram illustrating an exemplary configuration of an ultrasound diagnosis apparatus 100 according to the first embodiment. FIG. 2 is a block diagram illustrating an exemplary configuration of a medical information processing apparatus 200 according to the first embodiment. In the first embodiment, an example will be explained in which the ultrasound diagnosis apparatus 100 and the medical information processing apparatus 200 are communicably connected via a network (NW); however, possible embodiments are not limited to this example. For instance, the ultrasound diagnosis apparatus 100 and the medical information processing apparatus 200 are able to exchange information with each other via a storage medium, a removable external storage device, or the like, without the mediation of the network (NW).

First, a configuration of the ultrasound diagnosis apparatus 100 illustrated in FIG. 1 will be explained. As illustrated in FIG. 1, the ultrasound diagnosis apparatus 100 according to the first embodiment includes an ultrasound probe 101, an input interface 102, a display device 103, an electrocardiograph 104, and an apparatus main body 105. The ultrasound probe 101, the input interface 102, the display device 103, and the electrocardiograph 104 are communicably connected to the apparatus main body 105.

The ultrasound probe 101 includes a plurality of transducer elements. Each of the transducer elements is configured to generate an ultrasound wave on the basis of a drive signal supplied thereto from transmission and reception circuitry 110 included in the apparatus main body 105. Further, the ultrasound probe 101 is configured to receive reflected waves from a patient X and to convert the reflected waves into electrical signals. Further, the ultrasound probe 101 includes a matching layer provided for the transducer elements, as well as a backing member or the like that prevents the ultrasound waves from propagating rearward from the transducer elements. The ultrasound probe 101 is detachably connected to the apparatus main body 105.

When an ultrasound wave is transmitted from the ultrasound probe 101 to the patient X, the transmitted ultrasound wave is repeatedly reflected on a surface of discontinuity of acoustic impedances at a tissue in the body of the patient X and is received as reflected-wave signals by the plurality of transducer elements included in the ultrasound probe 101. The amplitude of each of the received reflected-wave signals is dependent on the difference between the acoustic impedances on the surface of discontinuity on which the ultrasound wave is reflected. When a transmitted ultrasound pulse is reflected on the surface of a moving blood flow, a cardiac wall, or the like, the reflected-wave signal is, due to the Doppler effect, subject to a frequency shift, depending on a velocity component of the moving members with respect to the ultrasound wave transmission direction.

The input interface 102 is configured to receive input operations of various types of instructions and information from an operator. More specifically, the input interface 102 is configured to convert the input operation received from the operator into electrical signals and to output the electrical signals to processing circuitry 170 included in the apparatus main body 105. For example, the input interface 102 is realized by using a trackball, a switch button, a mouse, a keyboard, a touchpad on which an input operation is performed by touching the operation surface thereof, a touch screen in which a display screen and a touchpad are integrally formed, contactless input circuitry using an optical sensor, audio input circuitry, and/or the like. The input interface 102 does not necessarily have to include one or more physical operation component parts such as a mouse and a keyboard. For instance, possible examples of the input interface 102 include electrical signal processing circuitry configured to receive an electrical signal corresponding to an input operation from an external input device provided separately from the apparatus and to output the electrical signal to controlling circuitry.

The display device 103 is configured to display various types of information and images. More specifically, the display device 103 is configured to convert the data of information and images sent thereto from the processing circuitry 170, into display-purpose electrical signals and to output the electrical signals. For example, the display device 103 is realized by using a liquid crystal monitor, a Cathode Ray Tube (CRT) monitor, a touch panel, or the like. Possible output devices to be included in the ultrasound diagnosis apparatus 100 are not limited to the display device 103 and may be a speaker, for example. For instance, the speaker is configured to output predetermined audio such as a beep sound to notify the operator of a processing status of the apparatus main body 105.

The electrocardiograph 104 is configured to obtain an electrocardiogram (ECG) of the patient X, as a biological signal of the patient X. The electrocardiograph 104 is configured to transmit the obtained electrocardiogram to the apparatus main body 105. In the present embodiment, an example is explained in which the electrocardiograph 104 is used as a means for obtaining information related to cardiac phases of the heart of the patient X. However, possible embodiments are not limited to this example. For instance, the ultrasound diagnosis apparatus 100 may obtain the information related to cardiac phases of the heart of the patient X, by acquiring times of the second heart sound (S2) in a phonocardiogram or an Aortic Valve Close (AVC) time obtained by measuring an ejected blood flow from the heart using spectrum Doppler.

The apparatus main body 105 is an apparatus configured to generate ultrasound image data on the basis of the reflected-wave signals received by the ultrasound probe 101. The apparatus main body 105 illustrated in FIG. 1 is an apparatus capable of generating two-dimensional ultrasound image data on the basis of two-dimensional reflected-wave data received by the ultrasound probe 101. Further, the apparatus main body 105 is an apparatus capable of generating three-dimensional ultrasound image data on the basis of three-dimensional reflected-wave data received by the ultrasound probe 101.

As illustrated in FIG. 1, the apparatus main body 105 includes the transmission and reception circuitry 110, signal processing circuitry 120, image generating circuitry 130, an image memory 140, storage circuitry 150, a network (NW) interface 160, and the processing circuitry 170. The transmission and reception circuitry 110, the signal processing circuitry 120, the image generating circuitry 130, the image memory 140, the storage circuitry 150, the NW interface 160, and the processing circuitry 170 are communicably connected to one another.

The transmission and reception circuitry 110 includes a pulse generator, a transmission delay unit, a pulser, and the like and is configured to supply the drive signal to the ultrasound probe 101. The pulse generator is configured to repeatedly generate a rate pulse used for forming a transmission ultrasound wave at a predetermined rate frequency. Further, the transmission delay unit is configured to apply a delay time period that is required to converge the ultrasound waves generated from the ultrasound probe 101 into the form of a beam and to determine transmission directionality and that corresponds to each of the transducer elements, to each of the rate pulses generated by the pulse generator. Further, the pulser is configured to apply the drive signal (a drive pulse) to the ultrasound probe 101 with timing based on the rate pulses. In other words, by varying the delay time periods applied to the rate pulses, the transmission delay unit arbitrarily adjusts the transmission directions of the ultrasound waves transmitted from the surfaces of the transducer elements.

The transmission and reception circuitry 110 has a function that is able to instantly change the transmission frequency, the transmission drive voltage, and the like, for the purpose of executing a predetermined scan sequence on the basis of an instruction from the processing circuitry 170. In particular, the function to change the transmission drive voltage is realized by using linear-amplifier-type transmission circuitry of which the value can be instantly switched or by using a mechanism configured to electrically switch between a plurality of power source units.

The transmission and reception circuitry 110 includes a pre-amplifier, an Analog/Digital (A/D) converter, a reception delay unit, an adder, and the like and is configured to generate the reflected-wave data by performing various types of processes on the reflected-wave signals received by the ultrasound probe 101. The pre-amplifier is configured to amplify the reflected-wave signal for each of the channels. The A/D converter is configured to perform an A/D conversion on the amplified reflected-wave signals. The reception delay unit is configured to apply a delay time period required to determine reception directionality. The adder is configured to generate the reflected-wave data by performing an adding process on the reflected-wave signals processed by the reception delay unit. As a result of the adding process performed by the adder, reflected components of the reflected-wave signals that are from the direction corresponding to the reception directionality are emphasize, so that a comprehensive beam used in the ultrasound wave transmission and reception is formed on the basis of the reception directionality and the transmission directionality.

In this situation, it is possible to select a form of the output signal from the transmission and reception circuitry 110, from among various forms such as being a signal called a Radio Frequency (RF) signal including phase information or being amplitude information resulting from an envelope detecting process.

The signal processing circuitry 120 is configured to generate data (B-mode data) in which the signal intensity is expressed by a degree of brightness, by receiving the reflected-wave data from the transmission and reception circuitry 110 and performing a logarithmic amplification and an envelope detecting process, or the like thereon. Further, the signal processing circuitry 120 is configured to generate data (Doppler data) obtained by extracting moving member information such as velocity, dispersion, power, and the like with respect to multiple points, by performing a frequency analysis to obtain velocity information from the reflected-wave data received from the transmission and reception circuitry 110 and extracting a blood flow, a tissue, and a contrast agent echo component subject to the Doppler effect.

The signal processing circuitry 120 illustrated in FIG. 1 is capable of processing both two-dimensional reflected-wave data and three-dimensional reflected-wave data. In other words, the signal processing circuitry 120 is configured to generate two-dimensional B-mode data from two-dimensional reflected-wave data and to generate three-dimensional B-mode data from three-dimensional reflected-wave data. Further, the signal processing circuitry 120 is configured to generate two-dimensional Doppler data from two-dimensional reflected-wave data and to generate three-dimensional Doppler data from three-dimensional reflected-wave data.

The image generating circuitry 130 is configured to generate the ultrasound image data from the data generated by the signal processing circuitry 120. In other words, the image generating circuitry 130 is configured to generate two-dimensional B-mode image data in which the intensities of reflected-waves are expressed with degrees of brightness from the two-dimensional B-mode data generated by the signal processing circuitry 120. Further, the image generating circuitry 130 is configured to generate two-dimensional Doppler image data indicating the moving member information from the two-dimensional Doppler data generated by the signal processing circuitry 120. The two-dimensional Doppler image data is a velocity image, a dispersion image, a power image, or an image combining together any of these images. The image generating circuitry 130 is also capable of generating M-mode image data from time-series data of B-mode data on one scanning line generated by the signal processing circuitry 120. Further, from the Doppler data generated by the signal processing circuitry 120, the image generating circuitry 130 is also capable of generating a Doppler waveform in which velocity information of a blood flow or a tissue is plotted in time series.

In this situation, generally speaking, the image generating circuitry 130 converts (by performing a scan convert process) a scanning line signal sequence from an ultrasound scan into a scanning line signal sequence in a video format used by, for example, television and generates display-purpose ultrasound image data. More specifically, the image generating circuitry 130 generates the display-purpose ultrasound image data by performing a coordinate transformation process compliant with the ultrasound scanning mode used by the ultrasound probe 101. Further, as various types of image processing processes besides the scan convert process, the image generating circuitry 130 performs, for example, an image processing process (a smoothing process) to re-generate an average brightness value image, an image processing process (an edge enhancement process) that uses a differential filter inside an image, or the like, by using a plurality of image frames resulting from the scan convert process. Also, the image generating circuitry 130 combines text information of various types of parameters, scale graduations, body marks, and the like with the ultrasound image data.

Further, the image generating circuitry 130 performs a rendering process on volume data for the purpose of generating various types of two-dimensional image data used for displaying the volume data on the display device 103. Examples of the rendering process performed by the image generating circuitry 130 include a process of generating Multi Planar Reconstruction (MPR) image data from the volume data by implementing an MPR method. Also, other examples of the rendering process performed by the image generating circuitry 130 include a process of performing a "Curved MPR" process on the volume data and a process of performing a "Maximum Intensity Projection" process on the volume data. Further, other examples of the rendering process performed by the image generating circuitry 130 include a Volume Rendering (VR) process and a Surface Rendering (SR) process.

In other words, the B-mode data and the Doppler data are each ultrasound image data before the scan convert process. The data generated by the image generating circuitry 130 is the display-purpose ultrasound image data after the scan convert process. The B-mode data and the Doppler data may be referred to as raw data. The image generating circuitry 130 is configured to generate "two-dimensional B-mode image data and two-dimensional Doppler image data", which is display-purpose two-dimensional ultrasound image data, from "two-dimensional B-mode data and two-dimensional Doppler data" which is two-dimensional ultrasound image data before the scan covert process.

The image memory 140 is a memory configured to store therein the display-purpose image data generated by the image generating circuitry 130. Further, the image memory 140 is also capable of storing therein any of the data generated by the signal processing circuitry 120. After a diagnosis process, for example, the operator is able to invoke any of the B-mode data and the Doppler data stored in the image memory 140. The invoked B-mode data and Doppler data can serve as the display-purpose ultrasound image data after being routed through the image generating circuitry 130.

In this situation, the image generating circuitry 130 is configured to store, into the image memory 140, the ultrasound image data and the time at which an ultrasound scan was performed to generate the ultrasound image data, so as to be kept in correspondence with an electrocardiographic waveform transmitted thereto from the electrocardiograph 104. By referring to the data stored in the image memory 140, the processing circuitry 170 (explained later) is able to obtain the cardiac phases at the time of the ultrasound scan performed to generate the ultrasound image data.

The storage circuitry 150 is configured to store therein various types of data. For example, the storage circuitry 150 is configured to store therein control programs for performing ultrasound wave transmissions and receptions, image processing processes, and display processes, diagnosis information (e.g., patients' IDs, medical doctors' observations), diagnosis protocols, and various types of data such as various types of body marks and the like. Further, the storage circuitry 150 may also be used, as necessary, for saving therein any of the image data stored in the image memory 140, and the like. For example, storage circuitry 150 is realized by using a semiconductor memory element such as a Random Access Memory (RAM) or a flash memory, or a hard disk, an optical disk, or the like. Further, the data stored in the storage circuitry 150 may be transferred to an external device via the NW interface 160.

The NW interface 160 is configured to control communication performed between the apparatus main body 105 and an external device. More specifically, the NW interface 160 is configured to receive various types of information from the external device and to output the received information to the processing circuitry 170. For example, the NW interface 160 is realized by using a network card, a network adapter, a Network Interface controller (NIC), or the like.

The processing circuitry 170 is configured to control the entirety of processes performed by the ultrasound diagnosis apparatus 100. More specifically, on the basis of various types of setting requests input from the operator via the input interface 102 and various types of control programs and various types of data read from the storage circuitry 150, the processing circuitry 170 controls processes performed by the transmission and reception circuitry 110, the signal processing circuitry 120, and the image generating circuitry 130. Further, the processing circuitry 170 is configured to control the display device 103 so as to display any of the display-purpose ultrasound image data stored in the image memory 140 and the storage circuitry 150.

Further, the processing circuitry 170 is configured to execute an obtaining function 171, a tracking function 172, a determining function 173, and an output controlling function 174. The obtaining function 171 is an example of the obtaining unit. The tracking function 172 is an example of a tracking unit. The determining function 173 is an example of the determining unit. The output controlling function 174 is an example of an output controlling unit. Details of the processes performed by the obtaining function 171, the tracking function 172, the determining function 173, and the output controlling function 174 executed by the processing circuitry 170 will be explained later.

In this situation, for example, processing functions performed by the constituent elements of the processing circuitry 170 illustrated in FIG. 1, namely, the obtaining function 171, the tracking function 172, the determining function 173, and the output controlling function 174 are recorded in the storage circuitry 150 in the form of computer-executable programs. The processing circuitry 170 is a processor configured to realize the functions corresponding to the programs by reading and executing the programs from the storage circuitry 150. In other words, the processing circuitry 170 that has read the programs has the functions illustrated within the processing circuitry 170 in FIG. 1.

In the present embodiment, the example is explained in which the single piece of processing circuitry (i.e., the processing circuitry 170) realizes the processing functions described below; however, another arrangement is also acceptable in which processing circuitry is structured by combining together a plurality of independent processors, so that the functions are realized as a result of the processors executing the programs.

Next, a configuration of the medical information processing apparatus 200 illustrated in FIG. 2 will be explained. As illustrated in FIG. 2, the medical information processing apparatus 200 includes an input interface 201, a display device 202, a NW interface 203, storage circuitry 204, and processing circuitry 210.

The input interface 201 is configured to receive various types of instructions and operations to input information from an operator. More specifically, the input interface 201 is configured to convert an input operation received from the operator into an electrical signal and to output the electrical signal to the processing circuitry 210. For example, the input interface 201 is realized by using a trackball, a switch button, a mouse, a keyboard, a touchpad on which an input operation is performed by touching the operation surface thereof, a touch screen in which a display screen and a touchpad are integrally formed, contactless input circuitry using an optical sensor, audio input circuitry, and/or the like. In this situation, the input interface 201 does not necessarily have to include one or more physical operation component parts such as a mouse and a keyboard. For instance, possible examples of the input interface 201 include electrical signal processing circuitry configured to receive an electrical signal corresponding to an input operation from an external input device provided separately from the apparatus and to output the electrical signal to controlling circuitry.

The display device 202 is configured to display various types of information and images. More specifically, the display device 202 is configured to convert the data of information and images sent thereto from the processing circuitry 210, into display-purpose electrical signals and to output the electrical signals. For example, the display device 202 is realized by using a liquid crystal monitor, a Cathode Ray Tube (CRT) monitor, a touch panel, or the like. Possible output devices included in the medical information processing apparatus 200 are not limited to the display device 202 and may be a speaker, for example. For instance, the speaker outputs predetermined audio such as a beep sound to notify the operator of a processing status of the medical information processing apparatus 200.

The NW interface 203 is connected to the processing circuitry 210 and is configured to control communication performed between the medical information processing apparatus 200 and an external device. More specifically, the NW interface 203 is configured to receive various types of information from the external device and to output the received information to the processing circuitry 210. For example, the NW interface 203 is realized by using a network card, a network adapter, an NIC, or the like.

The storage circuitry 204 is connected to the processing circuitry 210 and is configured to store therein various types of data. For example, the storage circuitry 204 is realized by a semiconductor memory element such as a RAM or a flash memory, or a hard disk, an optical disk, or the like.

The processing circuitry 210 is configured to control operations of the medical information processing apparatus 200 in accordance with input operations received from the operator via the input interface 201. For example, the processing circuitry 210 is realized by using a processor.

Further, the processing circuitry 210 is configured to execute a learning function 211. Details of processes performed by the learning function 211 executed by the processing circuitry 210 will be explained later. The learning function 211 is an example of a learning unit.

Incidentally, for medical cases exhibiting wall motion abnormality in a global region, it is possible for any medical provider to easily identify the abnormality because the abnormality is reflected in the ejection fraction of the heart. In contrast, it is difficult for even experienced people to find local abnormality in the wall motion involved in ischemic heart diseases, which are examples of diseases having a high mortality rate. For example, multiple experienced people who look at waveform information of strain of the same patient can often have mutually-different opinions. For this reason, for the purpose of accurately making a diagnosis of local abnormality in the wall motion involved in an ischemic heart disease, various techniques have been proposed by which a feature value obtained from waveform information of strain is defined as an index value or by which an index image is displayed by mapping such index values.

For example, a Post-systolic Strain Index (PSI) and a Strain Imaging Diastolic Index (SI-DI) have been proposed, as index values related to Post-Systolic Shortening (PSS), a delay in systole (tardokinesis), or a delay in diastole (postischemic diastolic stunning), which are signs (ischemic memory) specific to an ischemic state. Further, because a site exhibiting wall motion abnormality has relatively small strain compared to healthy sites, using peak strain has been proposed. Further, to capture a delay in the wall motion in an end-systolic period caused by ischemia, using Dyssynchrony Imaging (DI) has been proposed by which a time at which strain reached a peak value is rendered in an image. Further, for the purpose of capturing a delay in systole itself caused in an early systolic period by ischemia or capturing tardokinesis involved in pre-stretch, using Activation Imaging (AI) has been proposed by which a time at which strain reached a predetermined threshold value is rendered in an image.

However, in actual clinical situations, various types of existing indices related to diagnoses of ischemia may not function very well. For example, absolute values of strain vary among patients (examined subjects) and among regions of the heart. It is therefore not robust to determine whether or not hypokinesis is present in the wall motion of the heart being active, by using a peak value. Further, signs similar to the ischemic memory such as the PSS may also be found in healthy people in some situations. For this reason, the PSI and the SI-DI, which are defined while a focus is placed on timing abnormality in the waveform information of strain, also have low degrees of specificity. Further, even with ischemic cases, it is not always possible to observe characteristic signs in each of the individual indices, which lowers the level of precision of the identifying process. Accordingly, it is difficult to use classification using a "certain index" based on strain for identifying local abnormality in the wall motion, with a high level of precision and in a robust manner.

Incidentally, although it is difficult even for experienced people to identify local abnormality in the wall motion, a certain group of experienced people (hereinafter, simply "certain experienced people") who are extremely more experienced than other experienced people are able to identify abnormality in the wall motion by looking at waveform information. In that situation, it is considered that the certain experienced people (human beings) are able to capture a relative difference in the waveform information of local strain compared to global wall motion. Existing index values are considered to be an approach to capture such a difference. However, as mentioned above, there is a limit to the existing approach by which signs of local abnormality in the wall motion are defined and identified from the waveform information of strain. Further, although the above explanation mentions strain as an example of the wall motion parameters, the same applies to situations where displacements are used.

To cope with this situation, the medical information processing apparatus 200 according to the first embodiment is configured to construct a trained model by performing machine learning that uses a plurality of pieces of waveform information (a group of waveform information) of wall motion parameters in a plurality of medical cases as input data and that uses abnormality determination results obtained by the certain experienced people identifying whether or not wall motion abnormality is present as correct answer data. Further, the ultrasound diagnosis apparatus 100 according to the first embodiment is configured to output abnormality determination results of a diagnosed patient (an examined subject), by inputting a group of waveform information of wall motion parameters of the diagnosed patient to the trained model constructed by the medical information processing apparatus 200.

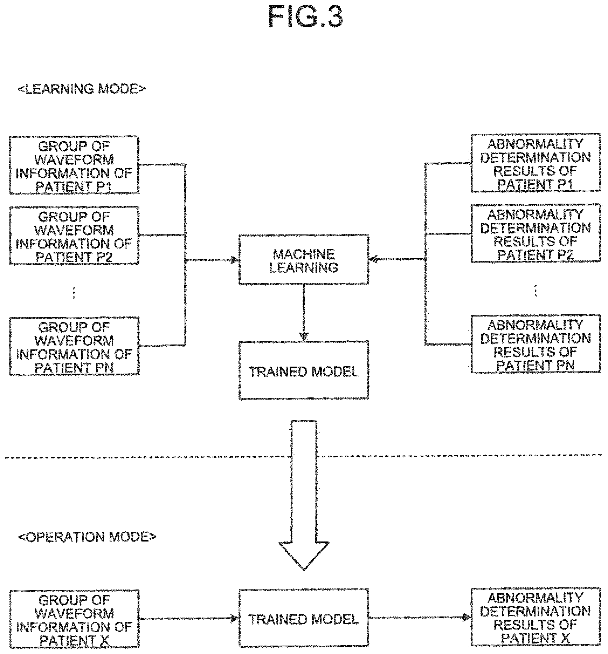

Processes performed by the ultrasound diagnosis apparatus 100 and the medical information processing apparatus 200 according to the first embodiment at the time of a learning process (hereinafter "learning mode") and at the time of an operation (hereinafter, "operation mode") will be explained, with reference to FIG. 3. FIG. 3 is a drawing illustrating the processes in the learning mode and the operation mode performed by the ultrasound diagnosis apparatus 100 and the medical information processing apparatus 200 according to the first embodiment.

As illustrated in the top section of FIG. 3, in the learning mode, the medical information processing apparatus 200 performs a machine learning process by using, for example, groups of waveform information and abnormality determination results of patients P1 to PN. In this situation, the groups of waveform information of the patients P1 to PN include a plurality of pieces of waveform information indicating time-series changes in wall motion parameters in a plurality of local regions and in a global region. Further, the abnormality determination results of the patients P1 to PN include abnormality determination results with respect to the local regions. By performing this machine learning process, the medical information processing apparatus 200 constructs the trained model configured to receive an input of a group of waveform information of a patient and to output abnormality determination results of local regions. The trained model is forwarded to the ultrasound diagnosis apparatus 100 and stored in the storage circuitry 150.

Further, as illustrated in the bottom section of FIG. 3, in the operation mode, the ultrasound diagnosis apparatus 100 inputs a group of waveform information of the patient X who is a diagnosed patient to the trained model constructed by the medical information processing apparatus 200 and thus causes the trained model to output abnormality determination results of the patient X. Further, the ultrasound diagnosis apparatus 100 is configured to present the operator with the abnormality determination results of the patient X output from the trained model.

Further, in the present embodiment, the "local regions" refers to the regions that are local. The "global region" refers to the entire region. Further, the patient X may be referred to as a "first patient". A local region of the first patient may be referred to as a "first local region", whereas the global region of the first patient may be referred to as a "first global region". The patients P1 to PN may be referred to as "second patients". Further, a local region of the second patients may be referred to as a "second local region", and the global region of the second patients may be referred to as a "second global region".

Next, processing functions of the ultrasound diagnosis apparatus 100 and the medical information processing apparatus 200 according to the first embodiment will be explained. In the following sections, at first, the medical information processing apparatus 200 configured to generate the trained model will be explained. After that, the ultrasound diagnosis apparatus 100 configured to determine local abnormality in the wall motion by using the trained model will be explained.

In the medical information processing apparatus 200, the storage circuitry 204 is configured to store therein, with respect to the hearts of the patients P1 to PN, groups of waveform information of strain in a plurality of local regions and in a global region as well as abnormality determination results of the plurality of local regions. The groups of waveform information of the strain constitute an example of the plurality of pieces of medical information indicating the time-series changes in the wall motion parameters.

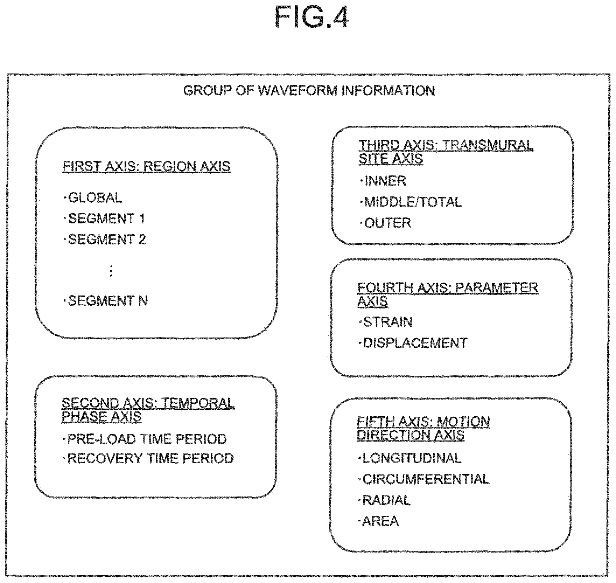

The groups of waveform information for the machine learning purpose according to the first embodiment will be explained with reference to FIG. 4. FIG. 4 is a drawing for explaining any of the groups of waveform information for the machine learning purpose according to the first embodiment.

As illustrated in FIG. 4, each of the groups of waveform information includes a plurality of pieces of waveform information. For example, the group of waveform information includes mutually-different types of information expressed on five axes, namely, first to fifth axes. In this situation, for the purpose of capturing the relative difference in the waveform information of local strain compared to the global wall motion, it is desirable to arrange the group of waveform information to include at least differences among regions. Further, the group of waveform information includes pieces of information that are of mutually-different types with respect to at least one selected from among the following: wall motion parameters; temporal phases; positions in the wall thickness direction (transmural site); and heart motion directions.

The first axis is a region axis indicating the differences among regions. For example, the first axis is expressed with "global" (the global region) and a plurality of segments 1, 2, 3, ... , and N (the local regions). The term "global" typically refers to the entire site subject to a speckle tracking analysis and may correspond, for example, to the entire left ventricle in a three-dimensional image or to the entirety of a region of interest in a two-dimensional cross-sectional image. Further, the "segments" are obtained by dividing the global region into sections by using a predetermined dividing method. Known examples of the dividing method include the 16-segment dividing method recommended by American Society of Echocardiography (ASE) and the 17-segment dividing method recommended by the American Heart Association (AHA). Further, the term "global" does not necessarily refer to the entire region of a certain site (e.g., the left ventricle) and may be any region larger than each of the local regions.

Further, as the waveform information of the global region, it is also possible to use waveform information indicating time-series changes in the volume of the lumen reflecting a cardiac function (the pumping function of the left ventricle). It is possible to calculate the waveform information of the volume of the lumen, on the basis of information of an endocardial surface from speckle tracking.

The second axis is a temporal phase axis indicating differences between temporal phases such as before and after either a load of stress echo or medical treatment. For example, when a stress echo test is performed, the second axis is expressed with a time period before a load (hereinafter, "pre-load time period") and a recovery time period. The pre-load time period is a temporal phase before an exercise load or a drug load is imposed on the heart of the patient, i.e., the temporal phase at rest. In contrast, the recovery time period is an arbitrary temporal phase after the load is imposed and before the patient returns to the state at rest and may be 5 minutes after the load or 10 minutes after the load, for example.

In this situation, the recovery time period is a temporal phase in which the ischemic memory appears. Because the period at rest serves as a temporal phase to be compared with the recovery time period, it is desirable to arrange the group of waveform information to include the two temporal phases in a pair. It should be noted, however, when no stress echo test is performed, the group of waveform information does not include the recovery time period and is used in the machine learning process only as information about the period at rest. Further, in the temporal phase during the load, because the patient is breathing hard and the heart rate increases, it is difficult to maintain the quality of the ultrasound image and the level of precision of the tracking. For this reason, it is desirable not to include the temporal phase during the load, as information on the temporal phase axis. Further, the second axis does not necessarily have to be the temporal phases before and after a load and may be, for example, temporal phases before and after medical treatment. In other words, the second axis may express differences between a temporal phase before treatment using a drug and a temporal phase after the treatment using the drug (e.g., a number of days or a number of months later) or may express differences between a temporal phase before surgery and a temporal phase after the surgery.

The third axis is transmural sites axis indicating differences among positions in the wall thickness direction. For example, the third axis is expressed with an inner layer, a middle layer, and an outer layer. For example, the inner layer denotes information on tracking points on an endocardium. The outer layer denotes information on tracking points on an endocardium. The middle layer denotes information at middle points between the tracking points on the endocardium and the tracking points on the endocardium.

In two-dimensional speckle tracking (2DST) schemes, because the spatial resolution of ultrasound images is sufficiently high, it is desirable to arrange the group of waveform information to include information about the inner, layer, the middle layer, and the outer layer. In contrast, in three-dimensional speckle tracking (3DST) schemes, because the spatial resolution of the ultrasound image is not sufficiently high, it is acceptable when the group of waveform information includes at least one selected from among: the inner layer, the middle layer, and the outer layer. However, because it is known that ischemic cardiac diseases can cause necrosis or myocardial ischemia in a tissue on the endocardial side more easily than in a tissue on the epicardial side, it is desirable to arrange the group of waveform information to include information about at least the inner layer.

Further, possible configurations of the third axis are not limited to the above examples. For instance, when the group of waveform information is represented by radial strain, the third axis may include an inner half, an outer half, and a total of the wall thickness. In other words, as the positions in the wall thickness direction, the group of waveform information includes either information about at least one selected from among the inner layer, the middle layer, and the outer layer; or information about at least one selected from among the inner half, the outer half, and the total of the wall thickness.

The fourth axis is a parameter axis indicating differences in the wall motion parameters. For example, the fourth axis is expressed with strain and displacements. It is sufficient when the group of waveform information includes, on the fourth axis, information about at least one of strain and displacements. However, to capture the wall motion with high sensitivity, it is desirable to arrange the fourth axis to include strain, which is an index of a change ratio based on a difference value (a length) between two displacements.

Further, because displacements are easily affected by movements of the entire heart (translation) and movements of the vicinity thereof (tethering), sensitivity is not very high. However, because displacements are not difference values, displacements are robust against noise. Because radial displacements (RD) are subject to visual recognition when experienced people look at movements on the endocardial surface, it is desirable to arrange the fourth axis to include radial displacements. Further, because Onishi (Onishi T, Uematsu M, Nanto S, Morozumi T, Watanabe T, Awata M, Iida O, Sera F, Nagata S. "Detection of diastolic abnormality by dyssynchrony imaging: correlation with coronary artery disease in patients presenting with visibly normal wall motion." Circ J. 2009 Jan;73(1):125-31.) suggests that longitudinal displacements (LD) have a possibility of contributing to detection of ischemic cardiac diseases, it is desirable to arrange the fourth axis to include longitudinal displacements.

The fifth axis is a motion direction axis indicating differences in motion directions of the heart. For example, in 3DST schemes, the fifth axis is expressed with a longitudinal direction, a circumferential direction, and a radial direction of the heart (myocardium), and an area size of a plane positioned parallel to the endocardial surface. For example, when the wall motion parameter is strain, the fifth axis is expressed with longitudinal strain (LS), circumferential strain (CS), and radial strain (RS). Further, the area size of the plane positioned parallel to the endocardial surface corresponds, as a change ratio thereof, to an Area Change (AC) ratio. Because the AC value expresses a change in a component (which includes a longitudinal direction, a circumferential direction, and a diagonal direction at the same time) that cannot be classified in any of the longitudinal, circumferential, and radial directions, the AC value is classified in the fifth axis.

When the wall motion parameter is displacements, it is desirable to arrange the fifth axis to be expressed with the LD and the RD explained above. Further, in 2DST schemes, it is desirable to arrange the fifth axis to be expressed with a longitudinal or circumferential direction and a radial direction.

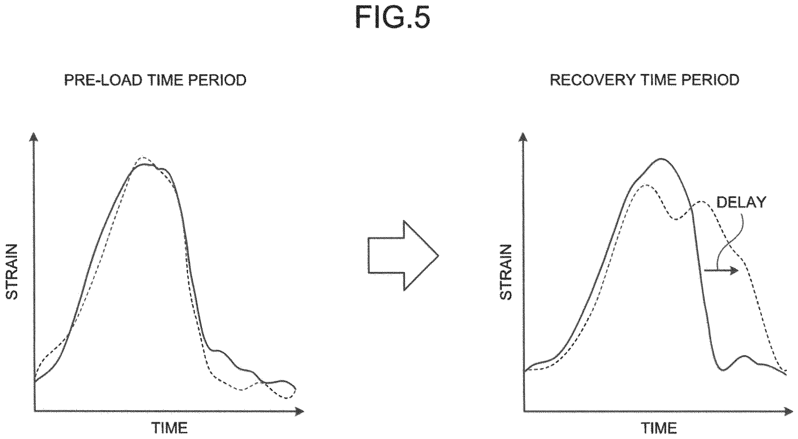

Examples of the group of waveform information according to the first embodiment will be explained, with reference to FIG. 5. FIG. 5 is a drawing for explaining the example of the group of waveform information according to the first embodiment. The left section of FIG. 5 illustrates waveform information of a pre-load time period (at rest), whereas the right section of FIG. 5 illustrates waveform information of a recovery time period (5 minutes after the load). In FIG. 5, the vertical axis corresponds to strain, whereas the horizontal axis corresponds to time. The solid line in FIG. 5 indicates strain in a segment corresponding to the anteroseptum, whereas the broken line indicates strain in a segment corresponding to the inferolateral part.

As indicated in the left section of FIG. 5, in the pre-load time period, the anteroseptum and the inferolateral part contract and expand with substantially the same timing as each other. In contrast, as indicated in the right section of FIG. 5, in the recovery time period, it is observed that expansion of the inferolateral part has a delay compared to that of the anteroseptum. The delay in the expansion of the anteroseptum is one of ischemic memory items. In other words, in FIG. 5, an ischemic abnormality in the wall motion is observed in the inferolateral part.

In the present example, the group of waveform information includes four pieces of waveform information illustrated in FIG. 5. However, although FIG. 5 includes the four pieces of waveform information for the sake of convenience in the preparation of the drawing, possible configurations of the group of waveform information are not limited to this example. The group of waveform information may include various types of waveform information having the differences on the first to the fifth axes explained above.

Each of the items in the group of waveform information explained above is information obtained by the wall motion analysis (the speckle tracking). Further, the group of waveform information is stored in the storage circuitry 204 in advance. The group of waveform information stored in the storage circuitry 204 may be calculated by the ultrasound diagnosis apparatus 100 or may be calculated by the medical information processing apparatus 200 having a function to execute the wall motion analysis. Further, the group of waveform information stored in the storage circuitry 204 does not necessarily have to be calculated by an apparatus provided in the same facility, but may be calculated by an apparatus installed in another facility.

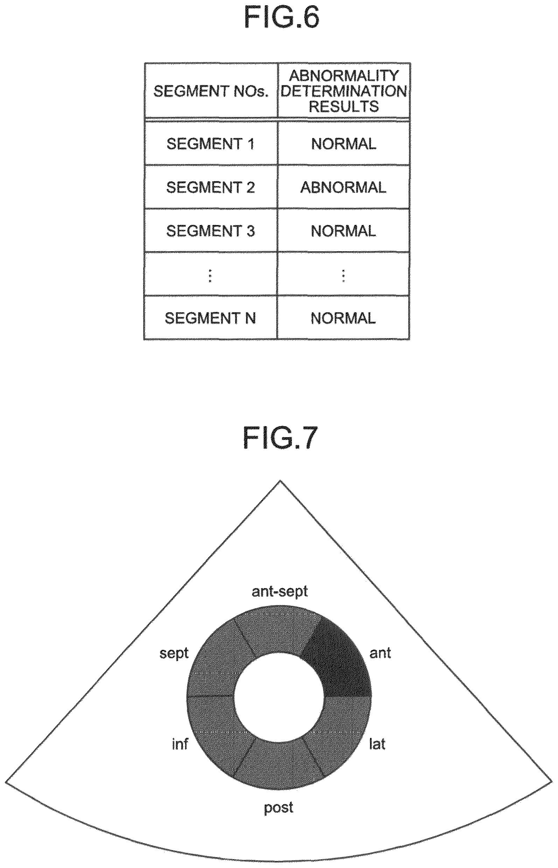

The abnormality determination results for the machine learning purpose according to the first embodiment will be explained, with reference to FIG. 6. FIG. 6 is a drawing for explaining the abnormality determination results for the machine learning purpose according to the first embodiment.

As indicated in FIG. 6, the abnormality determination results are information indicating whether each of the segments has wall motion abnormality or is normal. The abnormality determination results are information identified by the certain experienced people and are stored in the storage circuitry 204, in advance.

Although FIG. 6 illustrates the example in which the abnormality determination results are indicated as whether or not there is abnormality in the wall motion, possible embodiments are not limited to this example. For instance, the abnormality determination results may be expressed on multiple levels including a middle range. In that situation, for example, the abnormality determination results are expressed on the multiple levels where "normal" is expressed as "0", and the wall motion abnormality is expressed with the numeral "1 to 3 (the larger the value is, the more serious the abnormality is)".

As explained above, the storage circuitry 204 stores therein the groups of waveform information and the abnormality determination results. The groups of waveform information and the abnormality determination results described above are merely examples, and possible embodiments are not limited to those examples. For instance, each of the groups of waveform information may include any type of information, as long as it is possible to obtain the information by performing a publicly-known wall motion analysis.

In the medical information processing apparatus 200, the learning function 211 is configured to construct the trained model by performing the machine learning process, by using the groups of waveform information of the wall motion parameters in the plurality of local regions and in the global region, with respect to the hearts of the plurality of patients P1 to PN, and the abnormality determination results with respect to the plurality of local regions. The constructed trained model is stored in the storage circuitry 204. The groups of waveform information are each an example of the plurality of pieces of medical information indicating time-series changes in the wall motion parameters.

For example, the learning function 211 reads the groups of waveform information and the abnormality determination results of the plurality of patients P1 to PN from the storage circuitry 204. Further, the learning function 211 normalizes the time directions of the pieces of waveform information included in the groups of waveform information of the patients. For example, the learning function 211 normalizes the time directions of the pieces of waveform information by using one cardiac cycle period (1 R-R period) as 100%. In that situation, the learning function 211 generates (interpolates) the data (the wall motion parameters) so as to divide the one cardiac cycle period in units of 1% to 2%. As a result, the learning function 211 is able to express the pieces of waveform information as a one-dimensional vector space in which approximately 100 pieces of data are arranged in the one cardiac cycle period.

In the description above, the example is explained in which the time directions of the pieces of waveform information are normalized by regarding the one cardiac cycle period as 100%; however, possible embodiments are not limited to this example. It is possible to normalize the time directions on the basis of an arbitrary time period in the cardiac phases. For example, a sign specific to ischemic cardiac diseases is to recognize contraction after an end-systolic period or abnormal wall motion in an early diastole period on which a focus is placed by the PSS or the SI-DI. Accordingly, from the viewpoint of detecting ischemic cardiac diseases, it is also effective to perform the normalization by regarding the time period from an R-wave to an end-systolic period as 100%. The end-diastolic time period may be obtained from a time at which the volume of the lumen is at a minimum or an Aortic Valve Closure (AVC) time. In this situation, when the diastolic period is sufficiently long, it is desirable to cut off the time direction with a time period of approximately 200%.

Further, while it is possible to realize the learning function 211 by using a publicly-known machine learning engine, for example, a Support Vector Machine (SVM) may be applied. When an SVM is applied, the learning function 211 is configured to construct a correlation matrix among the pieces of waveform information with respect to each of the first to the fifth axes of the groups of waveform information serving as input data. Further, by performing a principal component analysis by using the correlation matrix, the learning function 211 generates the trained model by determining as many boundary planes (hyperplanes) as k that are in an eigenvector space and are ranked with the highest degrees of contribution to identifying normality and abnormality. The trained model is configured to identify whether the group of waveform information of the diagnosed patient is normal or abnormal (having wall motion abnormality), by inputting the group of waveform information to a discriminator based on the abovementioned hyperplanes in the eigenvector space.

To the machine learning process described above, it is also possible to apply a deep learning scheme (a neural network), which is another publicly-known machine learning engine. In this situation, the SVM scheme is specially designed to be able to obtain such hyperplanes that have maximum discriminatory margins for various types of input data. It is therefore possible to perform the identifying process even when the number of pieces of data used in the machine learning is not sufficiently large. In addition, as explained above, because the principal components (the eigenvalues ranked with the highest degrees of contribution) are used as the determination criterion between the normality and the abnormality, it is possible to provide a determination process that is relatively easy to understand for human beings. In contrast, in the deep learning scheme, the learning process is automatically performed when the waveform information serving as an input and the information about the abnormality determination results serving as correct answers are given, without the pre-processing of constructing the correlation matrix. However, the deep learning has a disadvantage where it is difficult for human beings to understand the determination criterion between normal results and abnormal results (the automatically-performed learning process, i.e., how weights to be applied to the neurons in different layers were designed.)

In the machine learning process (supervised learning) according to the present embodiment, because the abnormality determination results determined by the certain experienced people are used as the correct answer data, increasing the number of pieces of data used for the machine learning has a restriction. For this reason, as the machine learning engine of the present embodiment, it is desirable to use the SVM scheme. However, when the number of pieces of data used in the machine learning is sufficiently large, it is expected to be possible to achieve a high level of identifying capability with the deep learning scheme.

Next, the number of pieces of data used in the actual machine learning will be explained by using examples of 2DST and 3DST. The number of pieces of data used for the machine learning denotes the number of pieces (the number of types) of waveform information included in the group of waveform information of one patient. The number of pieces of data used for the machine learning explained below is merely an example and may vary depending on various types of conditions of the image taking process and the wall motion analysis.

First, an example using wall motion analysis results with 2DST will be explained. In this example, the 16-segment dividing method recommended by the ASE is commonly used, by which one cross-sectional plane is divided into 6 segments. Because a global region is added to the 6 segments, the number of region axes corresponds to 7 sites at minimum. Further, from the viewpoint of widely cover the left ventricle with a plurality of two-dimensional cross-sectional planes, a commonly-used combination is made up of 3 apical views (Apical 4-Chamber [A4C], Apical 2-Chamber [A2C], and Apical 3-Chamber [A3C]) and 1 short-axis (mid level) view. Accordingly, there are 7 sites $\times$ 4 cross-sectional planes = 28 sites. Alternatively, when the 17-segment dividing method recommended by the AHA is used, because there are 7 segments per cross-sectional plane, when a global region is added, there are 8 sites $\times$ 3 + 7 = 31 sites.

The wall motion parameters used for the 3 apical views are 2 parameters, namely LS and RD. Accordingly, there are 7 sites $\times$ 3 cross-sectional planes $\times$ 2 parameters = 42 parameters. Further, the number of wall motion parameters used for the 1 short-axis view is 3, namely RS, CS, and RD. Accordingly, there are 7 sites $\times$ 1 cross-sectional plane $\times$ 3 parameters = 21 parameters. Further, on each of the cross-sectional planes, the number of positions in the wall thickness direction is 3 (i.e., the inner layer, the middle layer, and the outer layer). Further, on each of the cross-sectional planes, the number of temporal phases is 2, namely the pre-load time period and the recovery time period. Accordingly, when results from the wall motion analysis with 2DST are used, the number of pieces of data used for the machine learning is calculated as $(42 + 21) \times 3 \times 2 = 378$ (types).

Next, an example using wall motion analysis results with 3DST will be explained. In this example, when the 16-segment dividing method recommended by the ASE is used, there are 16 segments + 1 global region = 17 sites. In contrast, when the 17-segment dividing method recommended by the AHA is used, there are 17 segments + 1 global region = 18 sites.

The wall motion parameters used for the 18 sites are 6 parameters, namely, LS, LD, RS, CS, RD, and AC, and 1 type represented by a capacity curve. Further, the number of positions in the wall thickness direction is 1 location, i.e., the inner layer. Further, in an example where the medical examination is not a stress echo test, the number of temporal phases is 1, i.e., a time period at rest. Accordingly, when results from the wall motion analysis with 3DST are used, the number of pieces of data for the machine learning is calculated as $18 \times (6 + 1) \times 1 \times 1 = 126$ (types).

It is also possible to apply both 2DST and 3DST to the same medical case (patient). Accordingly, when the numbers in the above two examples are added together, the number of pieces of data for the machine learning is calculated as 378 + 126 = 504 (types).

In other words, the number of pieces of data used for the machine learning is approximately in the range of hundreds to one thousand at maximum, depending on various types of conditions of the image taking process and the wall motion analysis. Further, the pieces of waveform information are expressed as a one-dimensional space in which approximately 100 pieces of data are arranged. As a result, the pieces of data used for the machine learning according to the present embodiment are structured as a vector space of approximately $1,000 \times 100$ at maximum.

As explained above, the machine learning function 211 is configured to construct the trained model by performing the machine learning process on the basis of the group of waveform information with the pieces of data in a quantity structured as the vector space of approximately $1,000 \times 100$ at maximum, and the abnormality determination results determined by the certain experienced people. The constructed trained model is forwarded to the ultrasound diagnosis apparatus 100 and is stored into the storage circuitry 150.

Returning to the description of FIG. 1, in the ultrasound diagnosis apparatus 100, the obtaining function 171 is configured to obtain a plurality of pieces of medical image data (a group of medical image data) taken of the heart of the patient X in a time series. For example, the ultrasound diagnosis apparatus 100 acquires reflected-wave data by performing an ultrasound scan on a region (a space) including the heart of the patient X. Further, on the basis of the acquired reflected-wave data, the ultrasound diagnosis apparatus 100 generates a plurality of pieces of ultrasound image data (a group of ultrasound image data) in the time series. In this situation, the generated plurality of pieces of ultrasound image data are stored in the image memory 140. The obtaining function 171 is configured to read the plurality of pieces of ultrasound image data taken of the heart of the patient X, from the image memory 140.

By performing a tracking process on the plurality of pieces of medical image data, the tracking function 172 is configured to generate a plurality of pieces of waveform information indicating time-series changes in the wall motion parameters in a plurality of local regions and in a global region. For example, the tracking function 172 generates wall motion information by performing a wall motion analysis on the plurality of pieces of ultrasound image data of the patient X.

For example, the tracking function 172 sets an initial contour set with a plurality of structure points for ultrasound image data in an initial temporal phase. Further, by performing a tracking process including pattern matching while using the ultrasound image data in the initial temporal phase with ultrasound image data in the following temporal phase, the tracking function 172 tracks the positions of the plurality of structuring points in the plurality of pieces of ultrasound image data included in the group of ultrasound image data. Further, the tracking function 172 generates wall motion information indicating the motion of the cardiac wall of the patient X, on the basis of the positions of the plurality of structuring points in the plurality of pieces of ultrasound image data included in each group of ultrasound image data.

In this situation, the wall motion information of the patient X generated by the tracking function 172 includes the same types of information as those of the groups of waveform information used for constructing the trained model by the medical information processing apparatus 200. In other words, the tracking function 172 is capable of generating the group of waveform information including the mutually-different types of information on the five axes, namely, the first to the fifth axes. As a technique for performing the wall motion analysis, it is possible to use any publicly-known technique.

The determining function 173 is configured to generate abnormality determination results for the local regions of the patient X, by inputting the group of waveform information of the patient X to the trained model. For example, the determining function 173 reads the trained model stored in the storage circuitry 150. Further, the determining function 173 causes the trained model to output the abnormality determination results for the local regions of the patient X, by inputting the group of waveform information generated by the tracking function 172 to the read trained model. Subsequently, the determining function 173 is configured to send the abnormality determination results output from the trained model to the output controlling function 174.

The abnormality determination results output from the trained model include the same types of information as those of the abnormality determination results used for constructing the trained model. In other words, when the abnormality determination results used in the machine learning process are represented by binary information indicating whether there is wall motion abnormality or not, the abnormality determination results output from the trained model are binary information. In contrast, when the abnormality determination results used in the machine learning process are represented by information expressed with multiple levels, the abnormality determination results output from the trained model are information expressed with the multiple levels.

The output controlling function 174 is configured to cause the abnormality determination results to be output. For example, the output controlling function 174 causes the abnormality determination results to be displayed while being superimposed on at least one of an ultrasound image and a polar map.

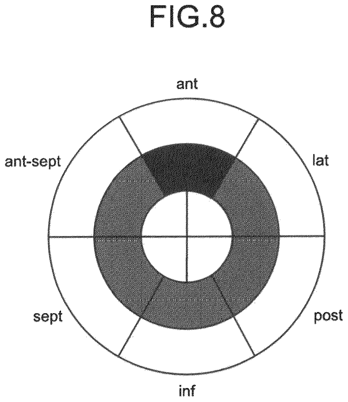

Examples of outputs by the output controlling function 174 according to the first embodiment will be explained with reference to FIGS. 7 and 8. FIGS. 7 and 8 are drawings for explaining the examples of the outputs by the output controlling function 174 according to the first embodiment. FIG. 7 illustrates an example with a short-axis view of the left ventricle. FIG. 8 illustrates a polar map corresponding to the short-axis view in FIG. 7. In FIGS. 7 and 8, a region determined to have wall motion abnormality are indicated with solid black, whereas regions determined to be normal are indicated with hatching. In FIGS. 7 and 8, "Ant-Sept" denotes the anteroseptum; "Ant" denotes the anterior wall; "Lat" denotes the lateral wall; "Post" denotes the posterior wall; "Inf" denotes the inferior wall; and "Sept" denotes the septum. Further, FIG. 7 illustrates the B-mode image in the background in a schematic manner.

As illustrated in FIG. 7, the output controlling function 174 causes the abnormality determination results to be displayed while being superimposed on the ultrasound image. More specifically, the output controlling function 174 realizes the display by assigning brightness values corresponding to the abnormality determination results to the positions corresponding to the segments in the short-axis view of the left ventricle. In the example in FIG. 7, the anterior wall has wall motion abnormality, while the other segments are normal.

As illustrated in FIG. 8, the output controlling function 174 causes the abnormality determination results to be displayed on the polar map. More specifically, the output controlling function 174 realizes the display by assigning brightness values corresponding to the abnormality determination results to the positions corresponding to the segments in the polar map. In the example in FIG. 8, the anterior wall has wall motion abnormality, while the other segments are normal.

In this manner, the output controlling function 174 causes the display device 103 to display the abnormality determination results. The examples in FIGS. 7 and 8 are merely examples, and possible embodiments are not limited to these examples. For instance, the output controlling function 174 is also capable of outputting the abnormality determination results of the segments in the form of a table (see FIG. 6) or a simple character string.

Further, the output controlling function 174 is capable of outputting, not only binary information indicating whether there is wall motion abnormality, but also continuous values including a middle range. The continuous values are each a value indicating which is closer between wall motion abnormality and normality. Thus, this method is considered to be a probabilistic output method (which may be referred to as "wall motion abnormality probability", for example). For instance, when a discriminator is used, there is an algorithm by which votes are cast on discriminated results (identified results) on the first to the fifth axes of the group of waveform information serving as input data, so as to eventually use results having larger votes as determination results.

Accordingly, it is possible to express the abnormality determination results as continuous values, by applying weights to the abnormality determination results according to a total number of votes cast on the pieces of waveform information. Further, when the deep learning scheme is used, it is possible to output a distribution image having continuous values that are spatially smooth, by performing a spatial averaging process on determination results of "0 (normal) / 1 (wall motion abnormality)" determined with respect to each of the segments or each of the positions in the wall thickness direction. Further, also when the abnormality determination results are expressed in multiple levels, it is possible to output approx continuous values.

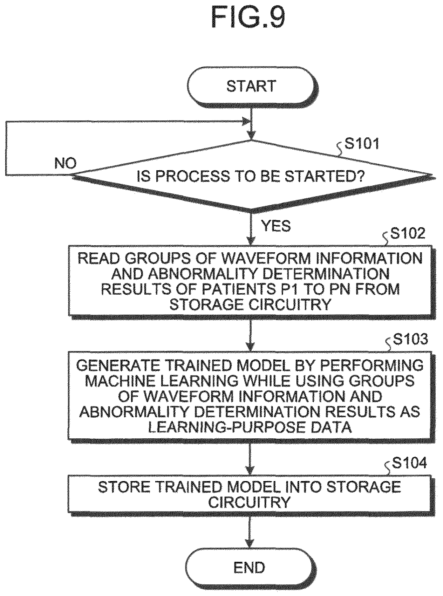

Next, a processing procedure performed by the medical information processing apparatus 200 according to the first embodiment will be explained, with reference to FIG. 9. FIG. 9 is a flowchart illustrating the processing procedure performed by the medical information processing apparatus 200 according to the first embodiment. The process illustrated in FIG. 9 is started, for example, when an instruction to start the machine learning process is received from the operator.

As illustrated in FIG. 9, for example, in the medical information processing apparatus 200, when having received an instruction to start the machine learning process from the operator, the processing circuitry 210 determines that the process is to be started (step S101: Yes). On the contrary, until such an instruction is received, the processing circuitry 210 is in a standby state (step S101: No).

Subsequently, the processing circuitry 210 reads the group of waveform information and the abnormality determination results of each of the patients P1 to PN from the storage circuitry 204 (step S102). Further, the processing circuitry 210 generates a trained model by performing the machine learning process by using the groups of waveform information and the abnormality determination results as learning-purpose data (step S103). After that, the processing circuitry 210 stores the generated trained model into the storage circuitry 204 (step S104), and ends the process.

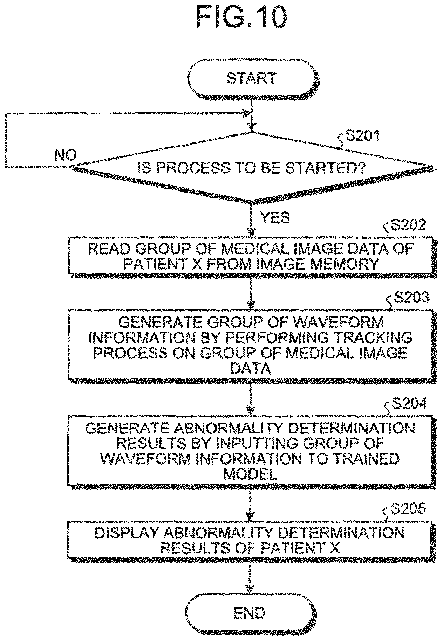

With reference to FIG. 10, a processing procedure performed by the ultrasound diagnosis apparatus 100 according to the first embodiment will be explained. FIG. 10 is a flowchart illustrating the proceeding procedure performed by the ultrasound diagnosis apparatus 100 according to the first embodiment. The process illustrated in FIG. 10 is started, for example, when an instruction to start the local wall motion abnormality determining process is received from the operator.

As illustrated in FIG. 10, for example, in the ultrasound diagnosis apparatus 100, when an instruction to start the local wall motion abnormality determining process is received from the operator, the processing circuitry 170 determines that the process is to be started (step S201: Yes). On the contrary, until such an instruction is received, the processing circuitry 170 is in a standby state (step S201: No) .

Subsequently, the processing circuitry 170 reads the group of medical image data of the patient X from the image memory 140 (step S202). Further, by performing the tracking process on the group of medical image data, the processing circuitry 170 generates a group of waveform information (step S203). By inputting the group of waveform information to the trained model, the processing circuitry 170 generate abnormality determination results (step S204). The processing circuitry 170 causes the abnormality determination results of the patient X to be displayed (step S205), and ends the process.

As explained above, in the ultrasound diagnosis apparatus 100 according to the first embodiment, the obtaining function 171 is configured to obtain the plurality of pieces of medical image data taken of the heart of the first patient in the time series. The tracking function 172 is configured to generate the plurality of pieces of first medical information indicating the wall motion parameters in the plurality of local regions and in the global region, by performing the tracking process on the plurality of pieces of medical image data. The determining function 173 is configured to generate the first abnormality determination result with respect to the local regions of the first patient, by inputting the plurality of pieces of first medical information to the trained model trained by using, with respect to the hearts of the plurality of second patients, the plurality of pieces of second medical information indicating the wall motion parameters in the plurality of local regions and in the global region and the abnormality determination results with respect to the local regions among the plurality of pieces of second medical information. The output controlling function 174 is configured to cause the first abnormality determination result to be output. With these arrangements, the ultrasound diagnosis apparatus 100 is able to identify local abnormality in the wall motion with a high level of precision and in a robust manner.

In the first embodiment, the example was explained in which the determining function 173 determines abnormality by using the waveform information of each of the plurality of local regions; however, possible embodiments are not limited to this example. For instance, by inputting the waveform information of at least one of the plurality of local regions focused on by a medical doctor and the waveform information of the global region to the trained model, the determining function 173 is also capable of determining abnormality of the local region. In other words, the determining function may be configured to determine abnormality in the first local region of the first patient, by inputting the plurality of pieces of first medical information of the first patient to the trained model trained by using, with respect to the hearts of the plurality of second patients, the plurality of pieces of second medical information indicating the time-series changes in the wall motion parameters in the second local region and in the second global region and the abnormality determination results with respect to the second local region.

Further, the obtaining function 171 may be configured to obtain the plurality of pieces of first medical information indicating the wall motion parameters in the first local region and in the first global region, with respect to the heart of the first patient. In that situation, the ultrasound diagnosis apparatus 100 does not necessarily have to include the tracking function 172. For example, the obtaining function 171 may obtain the plurality of pieces of first medical information generated by an external apparatus (another ultrasound diagnosis apparatus different from the ultrasound diagnosis apparatus 100 or a medical information processing apparatus) provided with the tracking function 172, from the external apparatus.

Second Embodiment

In a second embodiment, an example will be explained in which the operator is presented with publicly-known index values together with the abnormality determination results from the trained model. The reason is that it is difficult for the operator to understand the basis of the output results because the method using the trained model is complicated as to what was used as a basis of the determination. For this reason, the ultrasound diagnosis apparatus 100 according to the second embodiment is configured to present the operator with the publicly-known index values that are easy to understand for the operator as feedback of the abnormality determination results from the trained model, so as to be used as reference information for the abnormality determination results.

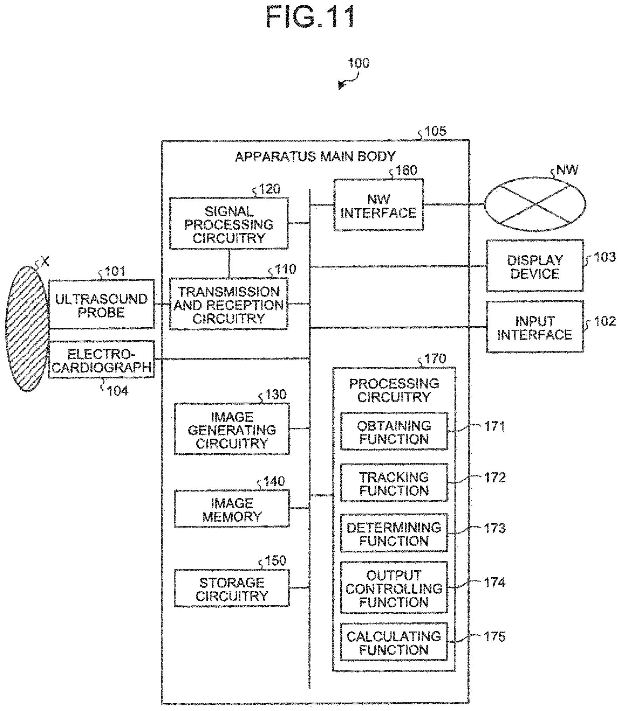

A configuration of the ultrasound diagnosis apparatus 100 according to the second embodiment will be explained, with reference to FIG. 11. FIG. 11 is a block diagram illustrating an exemplary configuration of the ultrasound diagnosis apparatus 100 according to the second embodiment. The ultrasound diagnosis apparatus 100 according to the second embodiment has almost the same configuration as that of the ultrasound diagnosis apparatus 100 illustrated in FIG. 1, except for the difference where the processing circuitry 170 further includes a calculating function 175. Thus, the second embodiment will be explained while a focus is placed on differences from the first embodiment. Some of the elements having the same functions as those explained in the first embodiment will be referred to by using the same reference characters as those in FIG. 1, and the explanations thereof will be omitted.

The calculating function 175 according to the second embodiment is configured to calculate one or more index values related to local abnormality in the wall motion. More specifically, the calculating function 175 is configured to calculate a plurality of types of index values with respect to the pieces of waveform information included in the group of waveform information of the patient X. For example, the calculating function 175 calculates an SI-DI and a PSI as the index values.

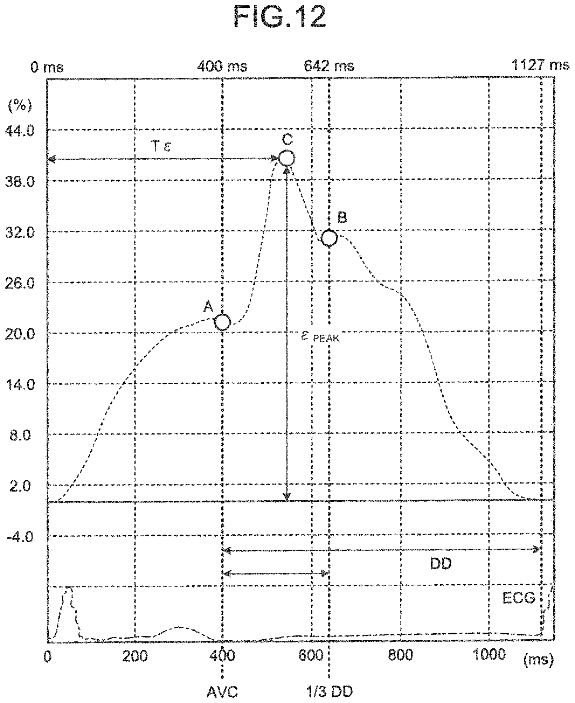

The index values calculated by the calculating function 175 according to the second embodiment will be explained, with reference to FIG. 12. FIG. 12 is a drawing for explaining the index values calculated by the calculating function 175 according to the second embodiment. In FIG. 12, the vertical axis corresponds to strain, whereas the horizontal axis corresponds to time.

As illustrated in FIG. 12, the calculating function 175 detects points A, B, and C from the waveform information. In the present example, point A corresponds to strain at an AVC time. Point B corresponds to strain at a time when one third of the diastolic period has elapsed since the AVC time. Point C corresponds to peak strain. Further, $T\varepsilon$ corresponds to the elapsed time period until reaching the peak strain.

Further, the calculating function 175 calculates the SI-DI and the PSI on the basis of the values (strain levels) at points A, B, and C illustrated in FIG. 12. For example, the calculating function 175 calculates the SI-DI by using Expression (1) presented below. Further, the calculating function 175 calculates the PSI by using Expression (2) presented below.

$$SI - DI = (A - B) / A \times 100 \tag{1}$$

$$PSI = (C - A) / C \times 100 \tag{2}$$

For example, the calculating function 175 is configured to perform a verification process on the various types of index values for the abnormality determination results obtained by the trained model. The calculating function 175 specifies, from among the plurality of types of index values, an index value being significant for the abnormality determination results, by performing a multivariate analysis. Alternatively, the calculating function 175 specifies an index value serving as the most principal component by performing a principal component analysis. Further, the calculating function 175 is configured to send information related to the specified index value to the output controlling function 174.

After that, the output controlling function 174 is configured to output the index value specified with respect to the abnormality determination results, together with the abnormality determination results. For example, for each of the pieces of waveform information, the output controlling function 174 outputs an index value that was significant for the abnormality determination results.

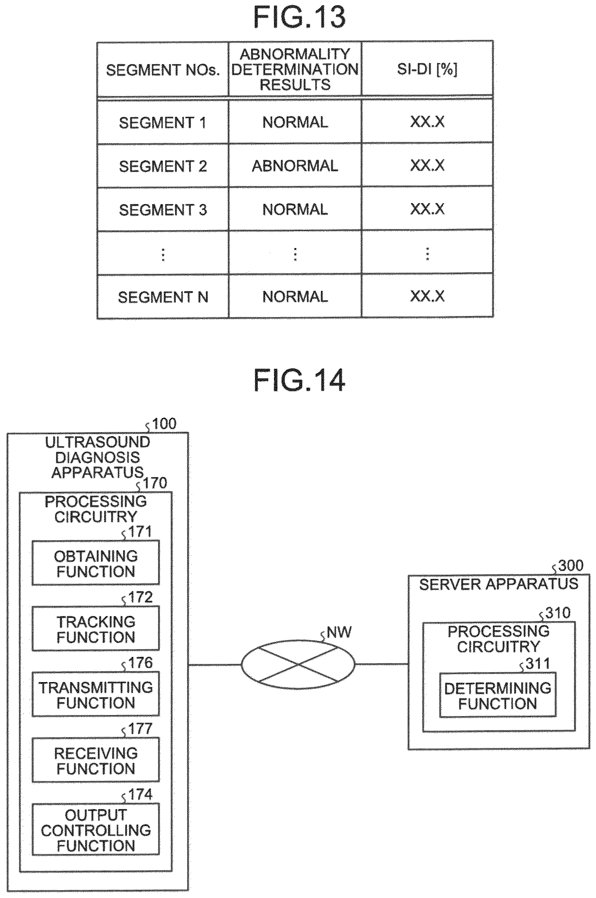

An example of the output by the output controlling function 174 according to the first embodiment will be explained, with reference to FIG. 13. FIG. 13 is a table for explaining the example of the output by the output controlling function 174 according to the first embodiment. FIG. 13 illustrates an example in which the SI-DI was specified as an index value significant for the abnormality determination results.

As illustrated in FIG. 13, the output controlling function 174 outputs SI-DI values in addition to the abnormality determination results illustrated in FIG. 6. The SI-DI values are displayed in correspondence with the segments. Further, the output controlling function 174 is capable of displaying, not only the index values, but also regression coefficients related to the plurality of index values used in the multivariate analysis, for example. Further, the output controlling function 174 may also display index values that were not significant.

The illustration in FIG. 13 is merely an example, and possible embodiments are not limited to this example. For instance, the output controlling function 174 is capable of generating and displaying an index image in which brightness values corresponding to the index values such as the SI-DI values are assigned.

As explained above, the ultrasound diagnosis apparatus 100 according to the second embodiment is configured to specify the index values that were either significant for the abnormality determination results or obtained as the principal components and to present the operator with the specified index values as the reference information, together with the abnormality determination results from the trained model. With this configuration, the ultrasound diagnosis apparatus 100 is able to assist the operator in interpretation of the abnormality determination results from the trained model.

In the description above, the example was explained in which the SI-DI and the PSI are calculated as the index values; however, possible embodiments are not limited to this example. For instance, the calculating function 175 may calculate an arbitrary index value, as long as the index value serves as an index value for local abnormality in the wall motion. For example, the calculating function 175 may calculate a peak strain level or a Diastolic Strain Ratio (DSR). In the present example, the DSR is a value obtained by calculating the same ratio as the SI-DI in Expression (1) for each of strain calculation points and further calculating an average of the calculation results among the calculation points included in a segment.

16

Other Embodiments

It is possible to carry out the present disclosure in various modes other than those described in the above embodiments.

Determining Process Performed By a Medical Information Processing System

The local wall motion abnormality determining process using the trained model described above may be executed by a medical information processing system. In that situation, the determining process is executed in the network (NW).

A configuration of the medical information processing system according to another embodiment will be explained with reference to FIG. 14. FIG. 14 is a block diagram illustrating an exemplary configuration of the medical information processing system according to the embodiment.

As illustrated in FIG. 14, the medical information processing system includes the ultrasound diagnosis apparatus 100 and a server apparatus 300. The ultrasound diagnosis apparatus 100 illustrated in FIG. 14 has, in principle, the same configuration as that of the ultrasound diagnosis apparatus 100 illustrated in FIG. 1, except that the processing circuitry 170 executes a transmitting function 176 and a receiving function 177. Further, the server apparatus 300 illustrated in FIG. 14 has, in principle, the same configuration as that of the medical information processing apparatus 200 illustrated in FIG. 2, except that processing circuitry 310 executes a determining function 311. The server apparatus 300 is an example of the medical information processing apparatus. The ultrasound diagnosis apparatus 100 and the server apparatus 300 are communicably connected to each other via the network (NW).

In the ultrasound diagnosis apparatus 100, because the processes performed by the obtaining function 171 and the tracking function 172 are, in principle, the same as the processes performed by the obtaining function 171 and the tracking function 172 illustrated in FIG. 1, the explanations thereof will be omitted. Further, the transmitting function 176 is configured to transmit the group of waveform information of the patient X generated by the tracking function 172, to the server apparatus 300.

In the server apparatus 300, the determining function 311 is configured to receive the group of waveform information transmitted thereto by the transmitting function 176. Further, the determining function 311 is configured to generate the abnormality determination results of the patient X by, inputting the received group of waveform information to the trained model. Because the processes performed by the determining function 311 are, in principle, the same as the processes performed by the determining function 173 illustrated in FIG. 1, the explanations thereof will be omitted. After that, the determining function 311 is configured to transmit the generated abnormality determination results of the patient X to the ultrasound diagnosis apparatus 100.

In the ultrasound diagnosis apparatus 100, the receiving function 177 is configured to receive the abnormality determination results of the patient X transmitted thereto by the determining function 311. Further, the output controlling function 174 is configured to cause the abnormality determination results of the patient X received by the receiving function 177 to be output. Because the processes performed by the output controlling function 174 are, in principle, the same as the processes performed by the determining function 173 illustrated in FIG. 1, the explanations thereof will be omitted.

To summarize, in the medical information processing system, the obtaining function 171 is configured to obtain the plurality of pieces of first medical information indicating the time-series changes in the wall motion parameters in the plurality of local regions and in the global region, with respect to the heart of the first patient. Further, the determining function 311 is configured to generate the first abnormality determination result with respect to the local regions of the first patient, by inputting the plurality of pieces of first medical information to the trained model trained by using, with respect to the hearts of the plurality of second patients, the plurality of pieces of second medical information indicating the time-series changes in the wall motion parameters in the plurality of local regions and in the global region and the abnormality determination results with respect to the local regions among the plurality of pieces of second medical information. Further, the output controlling function 174 is configured to cause the first abnormality determination result to be output. With these arrangements, the medical information processing system is able to perform the local wall motion abnormality determining process by using the trained model in the network (NW).

Determining Process Performed By the Medical Information Processing Apparatus

Further, the local wall motion abnormality determining process using the trained model described above may be performed by the medical information processing apparatus 200. For example, the medical information processing apparatus 200 corresponds to a terminal device operated by a medical doctor or a console device of another medical image diagnosis apparatus. In this situation, "another medical image diagnosis apparatus" denotes a medical image diagnosis apparatus other than the ultrasound diagnosis apparatus 100, such as a Magnetic Resonance Imaging (MRI) apparatus, an X-ray Computed Tomography (CT) apparatus, or the like.

In that situation, it is desirable to configure the medical information processing apparatus 200 to obtain the group of waveform information of the patient X from the ultrasound diagnosis apparatus 100. In other words, in the medical information processing apparatus 200, the processing circuitry 210 is configured to obtain the plurality of pieces of first medical information indicating the time-series changes in the wall motion parameters in the plurality of local regions and in the global region, with respect to the heart of the first patient. Further, the processing circuitry 210 is configured to generate the first abnormality determination result with respect to the local regions of the first patient, by inputting the plurality of pieces of first medical information to the trained model trained by using, with respect to the hearts of the plurality of second patients, the plurality of pieces of second medical information indicating the time-series changes in the wall motion parameters in the plurality of local regions and in the global region and the abnormality determination results with respect to the local regions among the plurality of pieces of second medical information. Further, the processing circuitry 210 is configured to output the first abnormality determination result.

Further, when the medical information processing apparatus 200 includes the same function as the tracking function 172, the medical information processing apparatus 200 is also capable of generating the group of waveform information of the patient X.

Using Medical Image Data Other Than Ultrasound Image Data

Further, in the embodiments above, the example is explained in which the present disclosure is applied to the group of ultrasound image data; however, possible embodiments are not limited to this example. For instance, it is possible to apply the present disclosure to any set of a plurality of pieces of medical image data in a time series, such as image data (e.g., magnetic resonance image data, CT image data) taken by any medical image diagnosis apparatus. In other words, it is possible to generate a group of waveform information by performing the wall motion analysis, as long as the data is a plurality of pieces of medical image data in a time series. Accordingly, it is possible to perform the determining process using the machine learning process and the trained model, by using the group of waveform information. In other words, the above embodiments are applicable not only to ultrasound diagnosis apparatuses, but also to other medical image diagnosis apparatuses.

Examples Other Than the Trained Model

In the description above, the example is explained in which the above embodiments are realized by the trained model; however, possible embodiments are not limited to this example. For instance, the ultrasound diagnosis apparatus 100 is capable of determining abnormality on the basis of a relative difference in the waveform information of local strain compared to the global wall motion.

In other words, in the ultrasound diagnosis apparatus 100, the obtaining function 171 is configured to obtain the plurality of pieces of first medical information indicating the time-series changes in the wall motion parameters in the first local region and in the first global region, with respect to the heart of the first patient. Further, the determining function 173 is configured to determine abnormality in the first local region on the basis of the plurality of pieces of first medical information.

In one example, the determining function 173 may be configured to detect the relative difference by comparing the waveform information of the global region with the waveform information of the local region. In this situation, the relative difference denotes, for example, a difference value in an arbitrary parameter (numerical values) such as integrated values obtained from the waveform information. The determining function 173 determines abnormality in the local region, on the basis of the detected difference.

The term "processor" used in the above explanations denotes, for example, a Central Processing Unit (CPU), a Graphics Processing Unit (GPU), or circuitry such as an Application Specific Integrated Circuit (ASIC) or a programmable logic device (e.g., a Simple Programmable Logic Device [SPLD], a Complex Programmable Logic Device [CPLD], or a Field Programmable Gate Array [FPGA]). The one or more processors realize the functions by reading and executing the programs saved in storage circuitry 150. Instead of saving the programs in the storage circuitry 150, it is also acceptable to directly incorporate the programs in the circuitry of the one or more processors. In that situation, the one or more processors realize the functions by reading and executing the programs incorporated in the circuitry thereof. The processors of the present embodiments do not each necessarily have to be configured as a single piece of circuitry. It is also acceptable to structure one processor by combining together two or more pieces of independent circuitry so as to realize the functions thereof. Further, two or more of the constituent elements illustrated in the drawings may be integrated into one processor so as to realize the functions thereof.

Further, the constituent elements of the apparatuses and the devices illustrated in the drawings are based on functional concepts. Thus, it is not necessary to physically configure the constituent elements as indicated in the drawings. In other words, specific modes of distribution and integration of the apparatuses and the devices are not limited to those illustrated in the drawings. It is acceptable to functionally or physically distribute or integrate all or a part of the apparatuses and the devices in any arbitrary units, depending on various loads and the status of use. Further, all or an arbitrary part of the processing functions performed by the apparatuses and the devices may be realized by a CPU and a program analyzed and executed by the CPU or may be realized as hardware using wired logic.

With regard to the processes explained in the above embodiments, it is acceptable to manually perform all or a part of the processes described as being performed automatically. Conversely, by using a method that is publicly known, it is also acceptable to automatically perform all or a part of the processes described as being performed manually. Further, unless noted otherwise, it is acceptable to arbitrarily modify any of the processing procedures, the controlling procedures, specific names, and various information including various types of data and parameters that are presented in the above text and the drawings.

Further, the medical image processing methods explained in the above embodiments may be realized by causing a computer such as a personal computer or a workstation to execute a medical image processing program prepared in advance. The medical image processing program may be distributed via a network such as the Internet. Further, the medical image processing program may be recorded on a computer-readable recording medium such as a hard disk, a flexible disk (FD), a Compact Disk Read-Only Memory (CD-ROM), a Magneto-Optical (MO) disk, a Digital Versatile Disk (DVD), or the like, so as to be executed as being read from the recording medium by a computer.

According to at least one aspect of the embodiments described above, it is possible to identify the local abnormality in the wall motion, with the high level of precision and in the robust manner.

While certain embodiments have been described, these embodiments have been presented by way of example only, and are not intended to limit the scope of the invention.

Various omissions, substitutions and changes in the form of the embodiments described herein may be made without departing from the scope of the inventions as defined by the appended claims.

**Claims**

1. An apparatus comprising:
   processing circuitry (170) configured to:

      obtain a plurality of pieces of first medical information indicating wall motion parameters in a first local region and in a first global region, with respect to a heart of a first patient;
      determine abnormality in the first local region on a basis of the plurality of pieces of first medical information using a trained model; and
      cause a determination result of the abnormality with respect to the first local region to be output,
      wherein the processing circuitry (170) is configured to calculate a plurality of types of index values with respect to each of the plurality of pieces of first medical information and to output one of the plurality of types of index values being significant or serving as the most principal component in the determination result of the abnormality with respect to the first local region, together with the determination result of the abnormality with respect to the first local region.

2. The apparatus according to claim 1, wherein the processing circuitry (170) is configured to determine the abnormality in the first local region of the first patient, by inputting the plurality of pieces of first medical information of the first patient to a trained model trained by using, with respect to hearts of a plurality of second patients, a plurality of pieces of second medical information indicating wall motion parameters in a second local region and in a second global region and abnormality determination results with respect to the second local region.

3. The apparatus according to claim 2, wherein

   the wall motion parameters include pieces of information that are of mutually-different types with respect to at least one selected from among: temporal phases before and after either a load or medical treatment; positions in a wall thickness direction; and heart motion directions, and
   the plurality of pieces of second medical information include information that is of same types as the plurality of pieces of first medical information.

4. The apparatus according to claim 3, wherein

   each of the first global region and the second global region corresponds to one of: an entire left ventricle in a three-dimensional image; and an entire region of interest in a two-dimensional cross-sectional image;

the first local region corresponds to at least one of a plurality of regions obtained by dividing the first global region into sections while using a predetermined dividing method; and

the second local region correspond to at least one of a plurality of regions obtained by dividing the second global region into sections while using a predetermined dividing method.

5. The apparatus according to any one of claim 3 or 4, wherein the wall motion parameters include information about at least one of strain and a displacement.

6. The apparatus according to any one of claims 3 to 5, wherein the temporal phases include information about at least one of: a time period before a load; and a recovery time period.

7. The apparatus according to any one of claims 3 to 6, wherein the positions in the wall thickness direction include either information about at least one selected from among an inner layer, a middle layer, and an outer layer; or information about at least one selected from among an inner half, an outer half, and a total of a wall thickness.

8. The apparatus according to any one of claims 3 to 7, wherein the motion directions include information about at least one selected from among: a longitudinal direction, a circumferential direction, a radial direction, and an area size of a plane positioned parallel to an inner myocardial membrane surface.

9. The apparatus according to any one of claims 3 to 8, wherein information about the wall motion parameters in the first global region and the second global region further includes information indicating time-series changes regarding changes in volume.

10. The apparatus according to any one of claims 2 to 9, wherein the trained model is constructed on a basis of one of a support vector machine and a neural network.

11. The apparatus according to claim 1, wherein the plurality of types of index values include at least a Strain Imaging Diastolic Index "SI-DI" and a Post-systolic Strain Index "PSI".

12. The apparatus according to any one of claims 1 to 11, wherein the processing circuitry (170) is configured to cause the determination result of the abnormality with respect to the first local region to be displayed while being superimposed on one of an ultrasound image and a polar map.

13. A computer program configured to cause a computer to perform processes of:

obtaining a plurality of pieces of first medical information indicating wall motion parameters in a first local region and in a first global region, with respect to a heart of a first patient;
determining abnormality in the first local region on a basis of the plurality of pieces of first medical information using a trained model;
causing a determination result of the abnormality with respect to the first local region to be output;
calculating a plurality of types of index values with respect to each of the plurality of pieces of first medical information; and
outputting one of the plurality of types of index values being significant or serving as the most principal component in the determination result of the abnormality with respect to the first local region, together with the determination result of the abnormality with respect to the first local region.

**Patentansprüche**

1. Vorrichtung, die Folgendes umfasst:
Verarbeitungsschaltungen (170), die zu Folgendem konfiguriert sind:

eine Vielzahl von Elementen von ersten medizinischen Informationen zu erlangen, die Wandbewegungsparameter in einem ersten örtlichen Bereich und in einem ersten umfassenden Bereich, in Bezug auf ein Herz eines ersten Patienten, angeben;
eine Anomalie in dem ersten örtlichen Bereich auf Grundlage der Vielzahl von Elementen von ersten medizinischen Informationen unter Verwendung eines trainierten Modells festzustellen; und
zu veranlassen, dass ein Feststellungsergebnis der Anomalie in Bezug auf den ersten örtlichen Bereich ausgegeben wird,
wobei die Verarbeitungsschaltungen (170) dafür konfiguriert sind, eine Vielzahl von Arten von Indexwerten in Bezug auf jedes von der Vielzahl von Elementen von ersten medizinischen Informationen zu berechnen und eine von der Vielzahl von Arten von Indexwerten, die signifikant sind oder als die hauptsächlichste Komponente bei dem Feststellungsergebnis der Anomalie in Bezug auf den ersten örtlichen Bereich dienen, zusammen mit dem Feststellungsergebnis der Anomalie in Bezug auf den ersten örtlichen Bereich auszugeben.

2. Vorrichtung nach Anspruch 1, wobei die Verarbeitungsschaltungen (170) dafür konfiguriert sind, die Anomalie in dem ersten örtlichen Bereich des ersten Patienten festzustellen durch Eingeben der Vielzahl von Elementen von ersten medizinischen Informationen des ersten Patienten in ein trainiertes Modell, das durch Verwenden, in Bezug auf Herzen einer Vielzahl von zweiten Patienten, einer Vielzahl von Elementen von zweiten medizinischen Informationen, die Wandbewegungsparameter in einem zweiten örtlichen Bereich und in einem zweiten umfassenden Bereich angeben, und von Anomalie-Feststellungsergebnissen in Bezug auf den zweiten örtlichen Bereich trainiert ist.

3. Vorrichtung nach Anspruch 2, wobei

die Wandbewegungsparameter Elemente von Informationen einschließen, die von wechselseitig unterschiedlichen Arten sind, in Bezug auf mindestens eines, ausgewählt unter Folgendem: zeitlichen Phasen vor und nach entweder einer Belastung oder einer medizinischen Behandlung; Positionen in einer Wanddickenrichtung; und Herzbewegungsrichtungen, und
die Vielzahl von Elementen von zweiten medizinischen Informationen Informationen einschließen, die von gleichen Arten sind wie die Vielzahl von Elementen von ersten medizinischen Informationen.

4. Vorrichtung nach Anspruch 3, wobei

jeder von dem ersten umfassenden Bereich und dem zweiten umfassenden Bereich einem von Folgendem entspricht: einer gesamten linken Herzkammer in einem dreidimensionalen Bild; und einem gesamten Bereich von Interesse in einem zweidimensionalen Querschnittsbild;
der erste örtliche Bereich mindestens einem von einer Vielzahl von Bereichen entspricht, die durch Teilen des ersten umfassenden Bereichs in Sektionen erhalten wird, während ein vorbestimmtes Teilungsverfahren verwendet wird; und
der zweite örtliche Bereich mindestens einem von einer Vielzahl von Bereichen entspricht, die durch Teilen des zweiten umfassenden Bereichs in Sektionen erhalten wird, während ein vorbestimmtes Teilungsverfahren verwendet wird.

5. Vorrichtung nach einem der Ansprüche 3 oder 4, wobei die Wandbewegungsparameter Informationen über mindestens eines von Deformation und einer Verlagerung einschließen.

6. Vorrichtung nach einem der Ansprüche 3 bis 5, wobei die zeitlichen Phasen Informationen über mindestens eines von Folgendem einschließen: einem Zeitraum vor einer Belastung; und einem Wiederherstellungszeitraum.

7. Vorrichtung nach einem der Ansprüche 3 bis 6, wobei die Positionen in der Wanddickenrichtung entweder Informationen über mindestens eines, ausgewählt unter einer inneren Schicht, einer mittleren Schicht und einer äußeren Schicht; oder Informationen über mindestens eines, ausgewählt unter einer inneren Hälfte, einer äußeren Hälfte und einer Gesamtheit einer Wanddicke, einschließen.

8. Vorrichtung nach einem der Ansprüche 3 bis 7, wobei die Bewegungsrichtungen Informationen über mindestens eines, ausgewählt unter Folgendem, einschließen: einer Längsrichtung, einer Umfangsrichtung, einer Radialrichtung und einer Flächengröße einer Ebene, die parallel zu einer inneren Myokardmembranoberfläche positioniert ist.

9. Vorrichtung nach einem der Ansprüche 3 bis 8, wobei Informationen über die Wandbewegungsparameter in dem ersten örtlichen Bereich und dem zweiten örtlichen Bereich ferner Informationen einschließen, die Zeitreihenveränderungen bezüglich von Veränderungen beim Volumen angeben.

10. Vorrichtung nach einem der Ansprüche 2 bis 9, wobei das trainierte Modell auf Grundlage eines von einer Support Vector Machine und einem neuronalen Netz konstruiert ist.

**11.** Vorrichtung nach Anspruch 1, wobei die Vielzahl von Arten von Indexwerten mindestens einen Diastolischen Deformationsbildgebungsindex "SI-DI" und einen Postsystolischen Deformationsindex "PSI" einschließt.

**12.** Vorrichtung nach einem der Ansprüche 1 bis 11, wobei die Verarbeitungsschaltungen (170) dafür konfiguriert sind, zu veranlassen, dass das Feststellungsergebnis der Anomalie in Bezug auf den ersten örtlichen Bereich angezeigt wird, während es auf einem von einem Ultraschallbild und eine Polar Map eingeblendet wird.

**13.** Rechnerprogramm, das dafür konfiguriert ist, zu veranlassen, dass ein Rechner folgende Prozesse durchführt:

Erlangen einer Vielzahl von Elementen von ersten medizinischen Informationen, die Wandbewegungsparameter in einem ersten örtlichen Bereich und in einem ersten umfassenden Bereich, in Bezug auf ein Herz eines ersten Patienten, angeben;
Feststellen einer Anomalie in dem ersten örtlichen Bereich auf Grundlage der Vielzahl von Elementen von ersten medizinischen Informationen unter Verwendung eines trainierten Modells;
Veranlassen, dass ein Feststellungsergebnis der Anomalie in Bezug auf den ersten örtlichen Bereich ausgegeben wird;
Berechnen einer Vielzahl von Arten von Indexwerten in Bezug auf jedes von der Vielzahl von Elementen von ersten medizinischen Informationen; und
Ausgeben einer von der Vielzahl von Arten von Indexwerten, die signifikant sind oder als die hauptsächlichste Komponente bei dem Feststellungsergebnis der Anomalie in Bezug auf den ersten örtlichen Bereich dienen, zusammen mit dem Feststellungsergebnis der Anomalie in Bezug auf den ersten örtlichen Bereich.

**Revendications**

**1.** Appareil comprenant :
des circuits de traitement (170) configurés pour :

obtenir une pluralité d'éléments de premières informations médicales indiquant des paramètres de mouvement de paroi dans une première région locale et dans une première région globale, relativement à un coeur d'un premier patient ;
déterminer une anomalie dans la première région locale sur la base de la pluralité d'éléments de premières informations médicales en utilisant un modèle formé ; et
provoquer l'émission en sortie d'un résultat de détermination de l'anomalie relativement à la première région locale,
dans lequel les circuits de traitement (170) sont configurés pour calculer une pluralité de types de valeurs d'index par rapport à chacun de la pluralité d'éléments de premières informations médicales et pour émettre en sortie un de la pluralité de types de valeurs d'indice qui sont significatives ou servent comme le composant le plus principal dans le résultat de détermination de l'anomalie relativement à la première région locale, ensemble avec le résultat de détermination de l'anomalie relativement à la première région locale.

**2.** Appareil selon la revendication 1, dans lequel les circuits de traitement (170) sont configurés pour déterminer l'anomalie dans la première région locale du premier patient, en entrant la pluralité d'éléments de premières informations médicales du premier patient dans un modèle formé, formé en utilisant, relativement à des coeurs d'une pluralité de deuxièmes patients, une pluralité d'éléments de deuxièmes informations médicales indiquant des paramètres de mouvement de paroi dans une deuxième région locale et dans une deuxième région globale et des résultats de détermination d'anomalie relativement à la deuxième région locale.

**3.** Appareil selon la revendication 2, dans lequel

les paramètres de mouvement de paroi comprennent des éléments d'informations qui sont de types réciproquement différents relativement à au moins un élément sélectionné parmi : des phases temporelles avant et après soit une charge soit un traitement médical ; des positions dans le sens d'épaisseur de paroi ; et des directions de mouvement de coeur, et
la pluralité d'éléments de deuxièmes informations médicales incluent des informations qui sont de mêmes types que la pluralité d'éléments de premières informations médicales.

**4.** Appareil selon la revendication 3, dans lequel

chacune de la première région globale et de la deuxième région globale correspond à un élément parmi : un ventricule gauche complet dans une image en trois dimensions ; et une région d'intérêt complète dans une image en coupe transversale en deux dimensions ;

la première région locale correspond à au moins une d'une pluralité de régions obtenues en divisant la première région globale en sections tout en utilisant un procédé de division prédéterminé ; et

la deuxième région locale correspond à au moins une d'une pluralité de régions obtenues en divisant la deuxième région globale en sections tout en utilisant un procédé de division prédéterminé.

5. Appareil selon l'une quelconque des revendications 3 ou 4, dans lequel les paramètres de mouvement de paroi incluent des informations concernant au moins un élément parmi une déformation et un déplacement.

6. Appareil selon l'une quelconque des revendications 3 à 5, dans lequel les phases temporelles incluent des informations concernant au moins un élément parmi : un laps de temps avant une charge ; et un laps de temps de récupération.

7. Appareil selon l'une quelconque des revendications 3 à 6, dans lequel les positions dans le sens d'épaisseur de paroi incluent soit des informations concernant au moins un élément sélectionné parmi une couche interne, une couche centrale, et une couche externe ; ou des informations concernant au moins un élément sélectionné parmi une moitié interne, une moitié externe, et un total d'une épaisseur de paroi.

8. Appareil selon l'une quelconque des revendications 3 à 7, dans lequel les directions de mouvement incluent des informations concernant au moins un élément sélectionné parmi : une direction longitudinale, une direction circonférentielle, une direction radiale, et une taille de superficie d'un plan positionné parallèlement à une surface de membrane myocardique interne.

9. Appareil selon l'une quelconque des revendications 3 à 8, dans lequel des informations concernant les paramètres de mouvement de paroi dans la première région globale et la deuxième région globale incluent en outre des informations indiquant des changements de série temporelle concernant des changements de volume.

10. Appareil selon l'une quelconque des revendications 2 à 9, dans lequel le modèle formé est construit sur la base d'un élément parmi une machine à vecteurs de support et un réseau neuronal.

11. Appareil selon la revendication 1, dans lequel la pluralité de types de valeurs d'indice incluent au moins un Indice Diastolique d'Imagerie de Déformation « SI-DI » et un Indice de Déformation Post-systolique « PSI ».

12. Appareil selon l'une quelconque des revendications 1 à 11, dans lequel les circuits de traitement (170) sont configurés pour provoquer l'affichage du résultat de détermination de l'anomalie par rapport à la première région locale tandis qu'il est superposée à une d'une image ultrasonore et d'une carte polaire.

13. Programme informatique configuré pour amener un ordinateur à effectuer des processus consistant à :

obtenir une pluralité d'éléments de première informations médicales indiquant des paramètres de mouvement de paroi dans une première région locale et dans une première région globale, relativement à un coeur d'un premier patient ;

déterminer une anomalie dans la première région locale sur la base de la pluralité d'éléments de premières informations médicales en utilisant un modèle formé ;

provoquer l'émission en sortie d'un résultat de détermination de l'anomalie relativement à la première région locale ;

calculer une pluralité de types de valeurs d'indice relativement à chacune de la pluralité d'éléments de premières informations médicales ; et

émettre en sortie un de la pluralité de types de valeurs d'indice qui sont significatives ou servent comme le composant le plus principal dans le résultat de détermination de l'anomalie relativement à la première région locale, ensemble avec le résultat de détermination de l'anomalie relativement à la première région locale.

# FIG.1

100

**APPARATUS MAIN BODY** 105

160
NW INTERFACE

120
SIGNAL PROCESSING CIRCUITRY

NW

DISPLAY DEVICE 103

X

101
ULTRASOUND PROBE

110
TRANSMISSION AND RECEPTION CIRCUITRY

INPUT INTERFACE 102

ELECTRO-CARDIOGRAPH
104

170
PROCESSING CIRCUITRY

130
IMAGE GENERATING CIRCUITRY

OBTAINING FUNCTION 171

TRACKING FUNCTION 172

140
IMAGE MEMORY

DETERMINING FUNCTION 173

150
STORAGE CIRCUITRY

OUTPUT CONTROLLING FUNCTION 174

# FIG.2

MEDICAL INFORMATION PROCESSING APPARATUS 200

NW

NW INTERFACE 203

DISPLAY DEVICE 202

INPUT INTERFACE 201

PROCESSING CIRCUITRY 210

LEARNING FUNCTION 211

STORAGE CIRCUITRY 204

# FIG.3

<LEARNING MODE>

GROUP OF WAVEFORM INFORMATION OF PATIENT P1

GROUP OF WAVEFORM INFORMATION OF PATIENT P2

GROUP OF WAVEFORM INFORMATION OF PATIENT PN

MACHINE LEARNING

TRAINED MODEL

ABNORMALITY DETERMINATION RESULTS OF PATIENT P1

ABNORMALITY DETERMINATION RESULTS OF PATIENT P2

ABNORMALITY DETERMINATION RESULTS OF PATIENT PN

<OPERATION MODE>

GROUP OF WAVEFORM INFORMATION OF PATIENT X

TRAINED MODEL

ABNORMALITY DETERMINATION RESULTS OF PATIENT X

# FIG.4

GROUP OF WAVEFORM INFORMATION

FIRST AXIS: REGION AXIS

·GLOBAL
·SEGMENT 1
·SEGMENT 2

⋮

·SEGMENT N

THIRD AXIS: TRANSMURAL SITE AXIS
·INNER
·MIDDLE/TOTAL
·OUTER

FOURTH AXIS: PARAMETER AXIS
·STRAIN
·DISPLACEMENT

SECOND AXIS: TEMPORAL PHASE AXIS
·PRE-LOAD TIME PERIOD
·RECOVERY TIME PERIOD

FIFTH AXIS: MOTION DIRECTION AXIS
·LONGITUDINAL
·CIRCUMFERENTIAL
·RADIAL
·AREA

FIG.5

# FIG.6

| SEGMENT NOs. | ABNORMALITY DETERMINATION RESULTS |
|---|---|
| SEGMENT 1 | NORMAL |
| SEGMENT 2 | ABNORMAL |
| SEGMENT 3 | NORMAL |
| ⋮ | ⋮ |
| SEGMENT N | NORMAL |

# FIG.7

# FIG.8

# FIG.9

START

S101

NO

IS PROCESS TO BE STARTED?

YES

S102

READ GROUPS OF WAVEFORM INFORMATION AND ABNORMALITY DETERMINATION RESULTS OF PATIENTS P1 TO PN FROM STORAGE CIRCUITRY

S103

GENERATE TRAINED MODEL BY PERFORMING MACHINE LEARNING WHILE USING GROUPS OF WAVEFORM INFORMATION AND ABNORMALITY DETERMINATION RESULTS AS LEARNING-PURPOSE DATA

S104

STORE TRAINED MODEL INTO STORAGE CIRCUITRY

END

# FIG.10

START

S201
NO — IS PROCESS TO BE STARTED?

YES

S202
READ GROUP OF MEDICAL IMAGE DATA OF PATIENT X FROM IMAGE MEMORY

S203
GENERATE GROUP OF WAVEFORM INFORMATION BY PERFORMING TRACKING PROCESS ON GROUP OF MEDICAL IMAGE DATA

S204
GENERATE ABNORMALITY DETERMINATION RESULTS BY INPUTTING GROUP OF WAVEFORM INFORMATION TO TRAINED MODEL

S205
DISPLAY ABNORMALITY DETERMINATION RESULTS OF PATIENT X

END

# FIG.11

100

APPARATUS MAIN BODY 105

160
NW INTERFACE

120
SIGNAL PROCESSING CIRCUITRY

NW

X

101
ULTRASOUND PROBE

110
TRANSMISSION AND RECEPTION CIRCUITRY

DISPLAY DEVICE 103

INPUT INTERFACE 102

ELECTRO-CARDIOGRAPH
104

170
PROCESSING CIRCUITRY

130
IMAGE GENERATING CIRCUITRY

OBTAINING FUNCTION 171

TRACKING FUNCTION 172

140
IMAGE MEMORY

DETERMINING FUNCTION 173

150
STORAGE CIRCUITRY

OUTPUT CONTROLLING FUNCTION 174

CALCULATING FUNCTION 175

# FIG.12

# FIG.13

| SEGMENT NOs. | ABNORMALITY DETERMINATION RESULTS | SI-DI [%] |
|---|---|---|
| SEGMENT 1 | NORMAL | XX.X |
| SEGMENT 2 | ABNORMAL | XX.X |
| SEGMENT 3 | NORMAL | XX.X |
| ⋮ | ⋮ | ⋮ |
| SEGMENT N | NORMAL | XX.X |

# FIG.14

ULTRASOUND DIAGNOSIS APPARATUS 100

PROCESSING CIRCUITRY 170

OBTAINING FUNCTION 171

TRACKING FUNCTION 172

TRANSMITTING FUNCTION 176

RECEIVING FUNCTION 177

OUTPUT CONTROLLING FUNCTION 174

NW

SERVER APPARATUS 300

PROCESSING CIRCUITRY 310

DETERMINING FUNCTION 311

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005001769 A

**Non-patent literature cited in the description**

- **ONISHI T ; UEMATSU M ; NANTO S ; MOROZUMI T ; WATANABE T ; AWATA M ; IIDA O ; SERA F ; NAGATA S.** Detection of diastolic abnormality by dyssynchrony imaging: correlation with coronary artery disease in patients presenting with visibly normal wall motion. *Circ J.,* January 2009, vol. 73 (1), 125-31